(19) Europäisches Patentamt
European Patent Office
Office européen des brevets

(11) EP 2 064 280 B1

(12) # EUROPEAN PATENT SPECIFICATION

(45) Date of publication and mention of the grant of the patent:
**20.04.2011 Bulletin 2011/16**

(21) Application number: **08773963.7**

(22) Date of filing: **10.07.2008**

(51) Int Cl.:
***C08K 5/14*** *(2006.01)*

(86) International application number:
**PCT/EP2008/005641**

(87) International publication number:
**WO 2009/007118 (15.01.2009 Gazette 2009/03)**

(54) **UNSATURATED POLYMER COMPOSITION**

UNGESÄTTIGTE POLYMERZUSAMMENSETZUNG

COMPOSITION DE POLYMÈRE INSATURÉ

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **12.07.2007 EP 07112305**

(43) Date of publication of application:
**03.06.2009 Bulletin 2009/23**

(73) Proprietor: **Borealis Technology OY**
**06101 Porvoo (FI)**

(72) Inventors:
- **SMEDBERG, Annika**
  **S-471 61 Myggenäs (SE)**
- **GUSTAFSSON, Bill**
  **S-444 41 Stenungsund (SE)**
- **NILSSON, Daniel**
  **S-415 12 Göteborg (SE)**

(74) Representative: **Campbell, Neil Boyd**
**Dehns**
**St Bride's House**
**10 Salisbury Square**
**London**
**EC4Y 8JD (GB)**

(56) References cited:

| | |
|---|---|
| **EP-A- 0 370 551** | **EP-A- 0 943 650** |
| **EP-A- 0 970 975** | **EP-A- 1 925 466** |
| **WO-A-01/66628** | **WO-A-02/44265** |
| **WO-A-91/16716** | **WO-A-99/23164** |
| **WO-A-99/27015** | **US-A- 3 578 647** |

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 064 280 B1

## Description

### Field of invention

The invention relates to an unsaturated polymer composition comprising a free radical generating agent, a process for modifying such an unsaturated polymer composition (e.g. to crosslink it), to modified polymer compositions, preferably cross-linked polymer compositions, to an article, preferably wire or cable, comprising said polymer composition, e.g. modified polymer composition, to a process for preparing an article, preferably a wire or cable and to the use of said polymer composition in one or more layers of a wire or cable..

### Background art

It is known to use free radical generating agents to modify a product, such as a polymer composition via a radical reaction.

Free radical agents are used e.g. to initiate (a) crosslinking in a polymer, i.a. primarily a formation of interpolymer crosslinks (bridges) by radical reaction, (b) grafting in a polymer, i.e. introduction of compounds to a polymer chain (to backbone and/or side chains) by radical reaction, and (c) visbreaking in a polymer, i.e. modification of melt flow rate (MFR) of a polymer by radical reaction. These polymer modifications are well known in the art.

When added to a polymer composition, free radical generating agents act by generating radicals, typically by decomposing to radicals, under conditions which enable the radical formation. The decomposed radicals initiate further radical reactions within a polymer composition. The resulting decomposition products of the free radical generating agent are typically a result of several reactions of the decomposition products of initial radical forming reaction. Said resulting decomposition products typically remain in the modified polymer and may include detrimental, undesired decomposition products.

Peroxides are very common free radical generating agents used i.a. in the polymer industry for said polymer modifications. The resulting decomposition products of peroxides may include volatile by-products. For example, dicumylperoxide, which is commonly used peroxide in polymer field, decomposes i.a. to methane, acetophenone and cumylalcohol during the radical formation step, e.g. during a crosslinking step. The formed gaseous methane ($CH_4$) is flammable, explosive and volatile and thus a risk in a working environment.

In wire and cable applications a typical cable comprises at least one conductor surrounded by one or more layers of polymeric materials. In some power cables, including medium voltage (MV), high voltage (HV) and extra high voltage (EHV) cables, said conductor is surrounded by several layers including an inner semiconductive layer, an insulation layer and an outer semiconductive layer, in that order. The cables are commonly produced by extruding the layers on a conductor. One or more of said layers are then typically crosslinked to improve i.a. deformation resistance at elevated temperatures, as well as mechanical strength and/or chemical resistance, of the layer(s) of the cable. The free radical generating agent, such as a peroxide, is typically incorporated to the layer material prior to the extrusion of the layer(s) on a conductor. After formation of the layered cable, the cable is then subjected to a crosslinking step to initiate the radical formation and thereby crosslinking reaction.

The decomposition products of the free radical forming agent remain mostly captured within the cable layer after crosslinking. This causes problems in view of the cable manufacturing process as well as in view of the quality of the final cable.

Accordingly, after crosslinking the cable must be cooled with great care to prevent the gaseous volatile decomposition products like methane forming voids within the polymer layer. These voids have typically an average diameter of between 10 to 100 μm. Partial discharges can take place in such voids within a cable that is subjected to an electrical field and thereby reduce the electrical strength of the cable.

The MV, HV and EHV power cables must have high layer quality in terms of safety during installation and in end use thereof. In service, volatile decomposition products in a cable resulting from a crosslinking step can create a gas pressure and thus cause defects in the shielding and in the joints. For example, when a cable is equipped with a metal barrier, then the gaseous products can exert a pressure, especially on the joints and terminations, whereby a system failure may occur.

For the above reasons the volatile decomposition products, such as methane e.g. where dicumylperoxide is used, are conventionally reduced to a minimum or removed after crosslinking and cooling step. Such removal step is generally known as a degassing step.

The degassing step is time and energy consuming and is thus a costly operation in a cable manufacturing process. Degassing requires large heated chambers which must be well ventilated to avoid the build-up of, for example, flammable methane and ethane. The cable, typically wound to cable drums, is normally degassed at elevated temperature in the range of 50-80°C, e.g. 60-70°C, for lengthy time periods. At these temperatures however, thermal expansion and softening of the insulation can occur and lead to undue deformation of the formed cable layers resulting directly in failures

of the cable. The degassing of MV, HV and EHV cables with high cable weight needs thus often be carried out at decreased temperatures.

Accordingly, there is a need to find new solutions to overcome the prior art problems.

The skilled man is also targetting cross-linking agents which are excellent cross-linkers. This invention concerns unsaturated polymers (further defined below) and the successful cross-linking of such polymers is made more complex by the presence of unsaturated groups i.a. in the polymer side chains. The present inventors have found peroxides which not only minimise the formation of volatile decomposition products but are also excellent cross-linkers for the unsaturated polymers which are the subject of this invention.

## Objects of the invention

One of the objects of the present invention is to provide an alternative process for modifying a polymer composition by using a free radical generating agent with superior properties.

A further object of the invention is to provide a polymer composition exhibiting excellent properties, such as high quality, useful for many end applications of polymers, i.a. for wire and cable applications.

Another object of the invention is to provide an article produced from said polymer composition, such as a cable which comprises one or more layers comprising said polymer composition, which article has highly advantageous properties, such as high quality and superior processability properties.

A further object of the invention is to provide a process for producing an article using said polymer composition, preferably a cable, as defined above, which process enables the preparation of high quality products with shorter production time and/or lower energy consumption.

The invention and further objects thereof are described and defined in details below.

## Description of the invention

The objects of the invention are solved by the polymer compositions, modified polymer compositions, end products and processes as defined below and in the claims.

## Polymer compositions of the invention

Viewed from one aspect the invention provides a polymer composition comprising

A) an unsaturated polymer, and
B) a free radical generating compound

- wherein the free radical forming agent is a compound of formula (I)

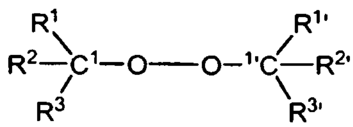

- $R^1$ and $R^{1'}$ are each independently H, substituted or unsubstituted saturated or partially unsaturated hydrocarbyl or substituted or unsubstituted aromatic hydrocarbyl;

- wherein each of said substituted or unsubstituted saturated or partially unsaturated hydrocarbyl or aromatic hydrocarbyl optionally comprises one or more heteroatoms;

- wherein said substituted or unsubstituted saturated or partially unsaturated hydrocarbyl include, preferably is selected from, (i) straight or branched chain saturated or partially unsaturated hydrocarbyls, (ii) straight or branched chain saturated or partially unsaturated hydrocarbyls which bear saturated or partially unsaturated cyclic hydrocarbyl and (iii) saturated or partially unsaturated cyclic hydrocarbyls;

- wherein each of said aromatic hydrocarbyl and said saturated or partially unsaturated cyclic hydrocarbyl is independently a monocyclic or multicyclic ring system; and

- wherein said substituted saturated or partially unsaturated hydrocarbyl or substituted aromatic hydrocarbyl comprise independently 1 to 4 substituents selected from a functional group, a saturated or partially unsaturated hydrocarbyl optionally bearing a functional group or aromatic hydrocarbyl optionally bearing a functional group;

- $R^2$, $R^{2'}$, $R^3$ and $R^{3'}$ are each independently H, substituted or unsubstituted saturated or partially unsaturated hydrocarbyl or substituted or unsubstituted aromatic hydrocarbyl;

- wherein each of said substituted or unsubstituted saturated or partially unsaturated hydrocarbyl or aromatic hydrocarbyl optionally comprises one or more heteroatoms;
- wherein said substituted or unsubstituted saturated or partially unsaturated hydrocarbyl include (i) straight or branched chain saturated or partially unsaturated hydrocarbyls, (ii) straight or branched chain saturated or partially unsaturated hydrocarbyls which bear saturated or partially unsaturated cyclic hydrocarbyl and (iii) saturated or partially unsaturated cyclic hydrocarbyls;
- wherein each of said aromatic hydrocarbyl and said saturated or partially unsaturated cyclic hydrocarbyl is independently a monocyclic or multicyclic ring system; and
- wherein said substituted saturated or partially unsaturated hydrocarbyl or substituted aromatic hydrocarbyl comprise independently 1 to 4 substituents selected from a functional group or a saturated or partially unsaturated hydrocarbyl optionally bearing a functional group or aromatic hydrocarbyl optionally bearing a functional group; or

-$R^2$ and $R^3$ together with the carbon atom ($C^1$) to which they are attached form an unsubstituted or substituted saturated or partially unsaturated carbocyclic ring moiety of 3 to 14 C-atoms, preferably 5-12 C atoms; an unsubstituted or substituted saturated or partially unsaturated heteroring moiety of 3 to 14 ring atoms comprising 1 to 6, preferably 1 to 4 heteroatoms, selected from O, N, P, S or Si; or an unsubstituted or substituted aromatic ring moiety of 3 to 14 C-atoms, preferably of 5-12 C atoms, optionally comprising 1 to 4 heteroatoms;

- wherein said carbocyclic ring, heteroring or aromatic ring system is optionally fused with another optionally substituted ring system having 4 to 14 ring atoms; and
- wherein said substituted carbocyclic ring, heteroring or aromatic ring system comprises 1 to 4 substituents selected independently from a functional group, or a saturated or partially unsaturated hydrocarbyl optionally bearing a functional group, or aromatic hydrocarbyl optionally bearing a functional group; or

-$R^{2'}$ and $R^{3'}$ together with the carbon atom ($C^{1'}$) to which they are attached form an unsubstituted or substituted saturated or partially unsaturated carbocyclic ring moiety of 3 to 14 C-atoms, preferably of 5-12 C atoms; an unsubstituted or substituted saturated or partially unsaturated heteroring moiety of 3 to 14 ring atoms comprising 1 to 6, preferably 1 to 4 heteroatoms, selected from O, N, P, S or Si; or unsubstituted or substituted aromatic ring moiety of 3 to 14 C-atoms, preferably moiety of 5 to 12 C atoms; optionally comprising 1 to 4 heteroatoms;

- wherein said carbocyclic ring, heteroring or aromatic ring system is optionally fused with another optionally substituted ring system having 4 to 14 ring atoms; and
- wherein said substituted carbocyclic ring, heteroring or aromatic ring system comprises 1 to 4 substituents selected independently from a functional group or a saturated or partially unsaturated hydrocarbyl optionally bearing a functional group or aromatic hydrocarbyl optionally bearing a functional group; or

- $R^2$ and $R^{2'}$ form together a bivalent substituted or unsubstituted saturated or partly unsaturated hydrocarbyl optionally containing 1 to 4 heteroatoms, wherein $R^2$ is linked to $C^1$ and $R^{2'}$, to $C^{1'}$, respectively, forming together with -$C^1$-O-O-$C^{1'}$- a substituted or unsubstituted saturated or partially unsaturated carbocyclic ring moiety of 3 to 14 C-atoms, preferably moiety of 5-12 C atoms, comprising optionally, in addition to said at least two O atoms, 1 to 4 further heteroatoms; wherein said carbocyclic ring or heteroring system is optionally fused with another ring system having 4-14 ring atoms;
and functional derivatives thereof;
- with the proviso that at least two of $R^1$, $R^2$ and $R^3$, and at least two of $R^{1'}$, $R^{2'}$ and $R^{3'}$, respectively, are other than H or methyl..

In a preferred embodiment of the invention the polymer composition comprises

A) an unsaturated polymer, and
B) a free radical generating compound

- wherein the polymer composition contains carbon-carbon double bonds in an amount of at least 0.05 or more, e.g. 0.1 or more, more preferably of 0.2 or more, and most preferably more than 0.37 carbon-carbon double bonds/1000 carbon atoms, and

- wherein the free radical forming agent (B) is a compound of formula (I) as hereinbefore described.

In a further preferred embodiment the invention provides a polymer composition comprising:

A) an unsaturated polymer, and
B) a free radical generating compound

- wherein the unsaturated polymer (A) contains carbon-carbon double bonds in an amount of 0.05 or more, e.g. 0.1 or more, more preferably of 0.2 or more, and most preferably more than 0.37 carbon-carbon double bonds/1000 carbon atoms, and

- wherein the free radical forming agent is a compound of formula (I) as herein before defined.

The polymer composition of the invention comprises at least one compound of formula (I) and hence may optionally comprise two or more compounds of formula (I) which are different. It may also comprise one or more other free radical generating agents.
Viewed from another aspect the invention provides a modified polymer composition in which the polymer composition as hereinbefore defined is crosslinked by initiating a radical reaction in the polymer composition.
The preferable embodiments and subgroups of the polymer composition of the invention, the components thereof and its modification, i.e. at least the crosslinking method, are described below generally for the invention and can be combined in any combination.

**Polymer composition**

The polymer composition of the invention comprises an unsaturated polymer (A). The expression "Polymer Composition" is used herein to mean the polymer composition of the invention. In a first embodiment, the amount of carbon-carbon (abbreviated to C-C) double bonds is measured based on the total amount of C-C double bonds present in the Polymer Composition as a whole. It is evident that at least the unsaturated polymer contains said C-C double bonds which contribute to the total amount of C-C double bonds. The Polymer Composition may optionally comprise further component(s) containing said C-C double bonds which then also contribute to the total amount of said C-C double bonds. In the first embodiment therefore, the C-C double bond content is thus measured on the composition as a whole not just on the unsaturated polymer component (A) thereof.
The carbon-carbon double bonds of the Polymer Composition preferably originate from vinyl groups, vinylidene groups or trans-vinylene groups, or from a mixture thereof, which are present in said Polymer Composition. The Polymer Composition does not necessarily contain all types of double bonds mentioned above. However, if it does, they all contribute to the "total amount of carbon-carbon double bonds" as defined above or below. The determination method for calculating the amounts of the carbon-carbon bonds in the above and below definitions is described under "Determination Methods".
The below defined preferable subgroups, further features, such as further properties or ranges thereof, and preferable embodiments apply generally to said Polymer Composition, to end applications and to any processes thereof, and can be combined in any combination.
The Polymer Composition contains preferably carbon-carbon double bonds in an amount of at least 0.05 or more, e.g. 0.1 or more, more preferably of 0.2 or more, and most preferably more than 0.37 carbon-carbon double bonds/1000 carbon atoms, e.g. at least 0.6/1000 carbon atoms, or preferably at least 0.8/1000 carbon atoms. The upper limit of the amount of carbon-carbon double bonds present in the Polymer Composition is not limited and may preferably be less of than 5.0/1000 carbon atoms, preferably less than 3.0/1000 carbon atoms, or more preferably less than 2.5/1000 carbon, or of up to 2.0/1000 carbon atoms.
The Polymer Composition comprises preferably at least vinyl groups as said carbon-carbon double bonds, which vinyl groups originate preferably from

i) a polyunsaturated comonomer,
ii) a chain transfer agent,
iii) an unsaturated low molecular weight compound which is, for example, a compound known as a crosslinking booster or a scorch retarder; or
iv) any mixture thereof.

In general, "vinyl group" means herein $CH_2$=CH- moiety which can be present in any of i) to iv) above.

The i) polyunsaturated comonomers and ii) chain transfer agents will be described below in relation to the unsaturated polymer of the Polymer Composition. The iii) unsaturated low molecular weight compound may be added into the Polymer Composition. The iii) unsaturated low molecular weight compound is preferably a crosslinking booster which is a compound containing at least 1, preferably at least 2, unsaturated groups, such as an aliphatic or aromatic compound, an ester, an ether, or a ketone, which contains at least 1, preferably at least 2, unsaturated group(s), such as a cyanurate, an isocyanurate, a phosphate, an orthoformate, an aliphatic or aromatic ether, or an allyl ester of benzene tricarboxylic acid. Examples of esters, ethers and ketones are compounds selected from general groups of diacrylates, triacrylates, tetraacrylates, triallylcyanurate, triallylisocyanurate, 3,9-divinyl-2,4,8,10-tetra-oxaspiro[5,5]-undecane (DVS) or triallyl trimellitate (TATM) or any mixtures thereof. The crosslinking booster can be added in an amount of less than 2.0 wt%, preferably of less than 1.5 wt%, more preferably of less than 1.0 wt%, and the lower limit thereof is typically at least 0.05 wt%, preferably of at least 0.1 wt%, based on the weight of the polymer composition.

Scorch retarders (SR) (further described below) as said iii) unsaturated low molecular weight component can also contribute to the total amount of C-C double bonds in the polymer compostion. Suitable scorch retarders include for example unsaturated dimers of aromatic alpha-methyl alkenyl monomers, such as 2,4-diphenyl-4-methyl-1-pentene, substituted or unsubstituted diphenylethylene, quinone derivatives, hydroquinone derivatives, monofunctional vinyl containing esters and ethers, or mixtures thereof. More preferably, the scorch retarder is selected from 2,4-diphenyl-4-methyl-1-pentene, substituted or unsubstituted diphenylethylene, or mixtures thereof. Preferably, the amount of scorch retarder is within the range of 0.005 to 2.0 wt.-%, more preferably within the range of 0.005 to 1.5 wt.-%, based on the weight of the Polymer Composition. Further preferred ranges are e.g. from 0.01 to 0.8 wt%, 0.03 to 0.75 wt%, 0.05 to 0.70 wt%, or 0.05 to 0.60 wt%, based on the weight of the Polymer Composition.

In one preferable embodiment, the C-C double bonds present in the Polymer Composition include vinyl groups and the total amount of said vinyl groups is, in the given preference order, of at least 0.05 or more, e.g. 0.1/1000 carbon atoms, preferably 0.2/1000 carbon atoms, 0.3/1000 carbon atoms, at least 0.4/1000 carbon atoms. In embodiments where higher vinyl content is desired the following ranges are preferable in the given preference order, at least 0.5/1000 carbon atoms, at least 0.6/1000 carbon atoms, or of at least 0.7/1000 carbon atoms. The upper limit of said vinyl groups is as defined above for carbon carbon double bonds. Accordingly, the total amount the vinyl groups, if present, contributes to the total amount of C-C double bonds present in the Polymer Composition. When the Polymer Composition contains vinyl groups from one or more of the sources (i) to (iv) as defined above, then the total amount of vinyl groups is the sum of vinyl groups amounting from each of one or more sources (i) to (iv) present in the Polymer Composition.

Preferably the unsaturated polymer of the Polymer Composition of the invention is a copolymer and comprises at least vinyl groups which originate from a polyunsaturated comonomer.

In a further preferable embodiment the $MFR_2$ of the Polymer Composition is from 0.01 to 50 g/10min, more preferably is from 0.1 to 20 g/10min, and most preferably is from 0.2 to 10g/10min.

The Polymer Composition is preferably crosslinkable and is highly suitable for producing crosslinkable articles, preferably one or more crosslinkable layers of a cable, which are subsequently crosslinked.

"Crosslinkable" is a well known expression and means that the Polyolefin Composition can be crosslinked, e.g. via radical formation, to form bridges i.a. amongst the polymer chains.

The free radical generating compound of formula (I) is preferably used as a crosslinking agent and is capable of generating radicals which can initiate a crosslinking reaction.

The Polymer Composition may contain also further additive(s). Such further additive(s) include:

- The above mentioned crosslinking booster(s) including the given specific compound(s), which can contribute to the crosslinking efficiency and/or to the total amount of C-C double bonds.

- Preferably one or more scorch retarders (SR) which are defined herein to be compounds that reduce the formation of scorch during extrusion of a polymer composition, at typical extrusion temperatures used, if compared to the same polymer composition extruded without said compound. As mentioned above scorch retardants can also contribute to the total amount of C-C double bonds in the polymer compostion. Preferred SR's and the usable amounts of SR are as given above.

- Further additive(s), such as antioxidant(s), stabiliser(s), and/or processing aid(s).

As an antioxidant, sterically hindered or semi-hindered phenol(s), aromatic amine(s), aliphatic sterically hindered amine(s), organic phosphate(s), thio compound(s), and mixtures thereof, can be mentioned. As further additive(s), flame retardant additive(s), water tree retardant additive(s), acid scavenger(s), inorganic filler(s) and voltage stabilizer(s) can be mentioned.

The Polymer Composition may additionally comprise further polymer component(s) including further unsaturated polymer(s) which are different from the at least one unsaturated polymer (component A), and polymer(s) that are not unsaturated.

In a preferred embodiment, the Polymer Composition does not contain further polymer components, i.e. it consists of the at least one unsaturated polymer as the sole polymer component. However, it is to be understood herein that the Polymer Composition may comprise further components such as above additives which may be added in a mixture with a carrier polymer, e.g. in so called master batch.

The Polymer Composition can be provided in the form of a powder or pellets in any shape and size including granules. Pellets can be produced, e.g. after polymerisation of the unsaturated polymer, in a well known manner using the conventional pelletising equipment, such as a pelletising extruder. Preferably, the Polymer Composition is provided in the form of pellets.

Preferably, the Polymer Composition comprises, more preferably consists of, the A) unsaturated polymer and the B) compound of formula (I), optionally together with further additive(s), and is in the form of pellets.

**Compound of formula (I) as a free radical generating agent (B)**

In a preferred embodiment the compound of formula (I) is not diphenylcyclohexyl peroxide.

In a further preferred embodiment, the compounds of the invention are subject to the proviso that when $R^2$ and $R^3$ together with the carbon atom ($C^1$) to which they are attached form a carbocyclic ring moiety of 3 to 14 C-atoms as defined above, and $R^{2'}$ and $R^{3'}$ together with the carbon atom ($C^{1'}$) to which they are attached form the carbocyclic ring moiety of 3 to 14 C-atoms as defined above, then $R^1$ or $R^{1'}$ cannot be an aromatic hydrocarbyl as defined.

The use of the compounds of formula (I) ensures that a reduced amount of a volatile decomposition products is formed during any cross-linking reaction compared to the prior art. Compounds of formula (I) are also believed to be ideal for the cross-linking of unsaturated polymers.

Preferably, the compound of formula (I) as defined above results in $CH_4$ content of less than 300 ppm (weight), preferably of less than 200 ppm (weight), preferably less than 100 ppm (weight), more preferably is without $CH_4$ as a decomposition product thereof, during an industrial process for generating free radicals, e.g. during a modification step of a polymer composition.

Generally, in above and below definitions the given values in ppm for methane and/or other volatile content are determined by gas chromatography from the obtained crosslinked polymer composition as such or from a crosslinked cable layer, depending on the definition, according to a method as described below under "GC-analysis protocol". Accordingly, the produced methane or other volatile content can equally be determined from a crosslinked polymer composition as such or from a crosslinked manufactured article thereof, as desired, each consisting of the polymer composition of the invention. The sample under the test is crosslinked using the test free radical generating agent in such an amount which results in a crosslinking degree expressed as gel content of 50 %, and preferably gel content of at least 50 %. The gel content (%) is measured according to ASTM D2765-01 Method A or B (depending on the nature of the sample). Such a crosslinked sample is then used for preparing the sample for volatile content measurement of GC-analysis protocol.

Without limiting to any theory, the terms "a decomposition product(s) thereof" or "a decomposition product of a free radical generating step" etc. as used above and below mean herein a by-product(s) formed during a free radical generating step, e.g. crosslinking step, and possibly also during the cooling step, by initiation of the free radical generating agent, as well known in the art. As an example methane may be one decomposition product which is an undesired decomposition product of the invention. Further decomposition products are specified below, which may not be desired in various embodiments of the invention.

Compounds of formula (I) are preferably without $CH_4$ as a decomposition product thereof. The absence of methane can be determined according to a method described below under "GC-analysis protocol". These compounds of the invention are suitable for embodiments where a high quality of a product which is modified with said compound are desired.

The term "without resulting in $CH_4$ as a decomposition product thereof" means that a compound of formula (I) of the present invention generates no methane, or in alternative terms does not decompose to the undesired volatile $CH_4$ by-product during a radical formation step in an industrial process.

The solution of the invention provides a new principal which is surprising and unobvious, since in the prior art there is no teaching or any indication to modify the free radical generating agent in order to avoid formation of $CH_4$ as a decomposing product during the free radical formation step in an industrial process. For example, in crosslinking

applications, the prior art has proposed merely solutions relating to balance the amount of free radical generating agent and the needed degree of crosslinking.

In one embodiment said compound of formula (I) of the invention results in reduced amount of or preferably does not decompose to low molecular weight compounds selected from (C1-C3) alkanes when generating free radicals, e.g. in industrial applications.

In another embodiment of the invention it is advantageous that said compound of formula (I) as a free radical generating agent results in reduced amount of or preferably is without (C1-C4) alkanes as decomposition products thereof when generating free radicals, e.g. in industrial applications.

In embodiments, wherein very high quality is required for the products modified by using a free radical agent, then it is preferable that said compound of formula (I) results in reduced amount of or is preferably without (C1-C6) alkanes as decomposition products thereof during a free radical forming step, e.g. in an industrial process.

The term "a free radical generating agent" is defined herein above or below to be any compound of formula (I) capable of generating radicals, e.g. in industrial applications, e.g. which can initiate a modification reaction in a polymer, such as a crosslinking, grafting or visbreaking reaction in a polymer.

Preferably compounds of formula (I) do not result in (i.e. are without) decomposition products, preferably hydrocarbon decomposition products, having a boiling point at atmospheric pressure of less than 50°C, preferably less than 80°C, or in some embodiments even less than 100°C may be desired. "Hydrocarbon" has the same meaning as given below for "hydrocarbyl" which represents a hydrocarbon as a monovalent substituent.

The terms used for defining the compounds of formula (I) are well known in the organic chemistry.

When the substituents are defined herein as "hydrocarbyl", "aromatic hydrocarbyl", "alkyl" etc. it is evident that they mean "a hydrocarbyl group", "an alkyl group" etc. For the avoidance of doubt, the term "hydrocarbyl" used herein does not encompass aromatic groups as is clear from the definitions used herein. The substituents are referred herein interchangeably as "radical" or "group", as known in the field.

Any hydrocarbyl group of the invention will preferably have up to 40C, atoms, preferably up to 30 C atoms, e.g. up to 20 C atoms, especially up to 12 carbon atoms. Some highly preferred hydrocarbyls may have 1 to 6 carbon atoms.

Alkyl groups, alkenyl groups or alkynyl groups defined in formula (I) and (V) and in preferable embodiments, and subgroups thereof as defined above below and claims, will preferably have up to 40C, atoms, preferably up to 30 C atoms, e.g. up to 20 C atoms. Some highly preferred alkyl groups may have 1 to 12 carbon atoms, more preferably may be methyl or have 6 to 12 carbon atoms.

Cyclic alkyl or cyclic alkenyl groups will preferably having up to 20 C atoms, especially up to 12 carbon atoms. Some highly preferred cyclic alkyl groups may have 3 to 8 carbon atoms. Preferred cyclic alkenyl groups may have 5 to 8 carbon atoms.

Aromatic hydrocarbyl groups may have up to 40C, atoms, preferably up to 30 C atoms, e.g. up to 20 C atoms, especially up to 12 carbon atoms. Some highly preferred aromatic hydrocarbyls may have 6 to 12 carbon atoms.

The expression "partially unsaturated" means that the moiety may comprise one or more double or triple bonds and include alkenyl radicals comprising at least one double bond and alkenyl radicals comprising at least one triple bond. In case of "partially unsaturated cyclic hydrocarbyl" there can be one or more double bonds in the ring systems meaning that the ring is non-aromatic to differentiate said "partially unsaturated" ring moieties from "aromatic rings" such as phenyl or pyridyl radicals.

"Hetero atoms" present in the moieties of the invention are selected from N, O, P, S or Si. Such moieties include e.g. hydrocarbyl or cyclic hydrocarbyl moieties containing one or more heteroatoms, as defined above and below, and to heteroring or heterocyclic ring moieties as defined above and below, which are understood to contain C-atoms in addition to the heteroatoms.

The expression "monocyclic" includes monocyclic ring systems, such as cyclopentyl, cyclohexyl, cycloheptyl or phenyl.

The expression "multicyclic" in turn means herein fused ring systems, such as naphthyl.

Unless otherwise defined herein, the term "carbocyclic" means substituted or unsubstituted saturated or partially unsaturated cyclic hydrocarbyl ring system; or substituted or unsubstituted aromatic hydrocarbyl ring system.

The term "functional group" as a substituent is well known expression and includes i.a. -OH, $-NR_2$, wherein each R is independently H or (C1-C12)alkyl; COR", wherein R" is i.a. H, (C1-C12)alkyl or $-NR_2$, wherein each R is as defined for $-NR_2$; COOR", wherein R" is as defined for -COR". A further functional group is a halogen, such as -F, -Cl or -I.

Other preferred functional groups include alkoxy, e.g. $OC_{1-12}$alkyl, nitro, thiol, thio$C_{1-12}$alkyl and CN.

The term "optional" means "may or may not be present", e.g. "optionally substituted" cover the possibilities that a substituent is present and or not present. The term "unsubstituted" naturally means that no substituent is present.

When $R^2$ and $R^3$ together with $C^1$ to which they are attached form a ring system as defined above, or respectively, $R^{2'}$ and $R^{3'}$ together with $C^{1'}$ to which they are attached form a ring system as defined above, then, as $C^1/C^{1'}$ are fully valenced, it is understood herein that any optional substituents or substituent(s) Z as used above and below means substituents linked to ring atom(s) other than $C^1$ and $C^{1'}$, respectively.

In compounds (I), when $R^2$ and $R^3$ form together with $C^1$ an aromatic ring as defined above, then $R^1$ is not present, and, respectively, when $R^{2'}$ and $R^{3'}$ form together with $C^{1'}$ an aromatic ring, as defined above, then $R^{1'}$ is not present. Preferably however $R^2$ and $R^3$ together with $C^1$ and $R^{2'}$ and $R^{3'}$ together with $C^{1'}$ do not form an aromatic ring.
By functional derivative of compounds of formula (I) means that at least one of $R^1$, $R^2$, $R^3$, $R^{1'}$, $R^{2'}$, $R^{3'}$ is in form of functional derivative. The term "functional derivative" includes i.a. esters and salts of compounds of formula (I), in particular esters and salts of substituents $R^1$, $R^2$, $R^3$, $R^{1'}$, $R^{2'}$, $R^{3'}$. Preferred compounds (I) are those, wherein $R^1$, $R^2$, $R^3$, $R^{1'}$ $R^{2'}$ $R^{3'}$ are as defined above or below are not functional derivatives thereof.
A further preferred subgroup of compounds of formula (I) is a compound of formula (V)

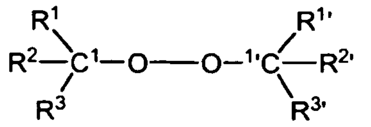

(V)

wherein the compounds are selected from any of the alternatives (i) to (iii):

(i) - $R^1$ and $R^{1'}$ are each independently H, substituted or unsubstituted saturated or partially unsaturated hydrocarbyl;

- wherein each of said substituted or unsubstituted saturated or partially unsaturated hydrocarbyl optionally comprises one or more heteroatoms;
- wherein said substituted or unsubstituted saturated or partially unsaturated hydrocarbyl include (i) straight or branched chain saturated or partially unsaturated hydrocarbyls, (ii) straight or branched chain saturated or partially unsaturated hydrocarbyls which bear saturated or partially unsaturated cyclic hydrocarbyl and (iii) saturated or partially unsaturated cyclic hydrocarbyls;
- wherein each of said saturated or partially unsaturated cyclic hydrocarbyl is independently a monocyclic or multicyclic ring system; and
- wherein said substituted saturated or partially unsaturated hydrocarbyl comprise independently 1 to 4 substituents selected from a functional group, a saturated or partially unsaturated hydrocarbyl optionally bearing a functional group or aromatic hydrocarbyl optionally bearing a functional group; and
- $R^2$, $R^{2'}$, $R^3$ and $R^{3'}$ are each independently as defined above for $R^1$ and $R^{1'}$;
or

(ii) - $R^1$ and $R^{1'}$ are each independently an optionally substituted, preferably unsubstituted, monocyclic (C5-C7)aryl, preferably phenyl,

- wherein said substituted monocyclic (C5-C7)aryl comprises independently 1 to 4 substituents selected from a functional group, a saturated or partially unsaturated hydrocarbyl optionally bearing a functional group or aromatic hydrocarbyl optionally bearing a functional group; and

$R^2$ and $R^{2'}$ are same and are both methyl; and
$R^3$ and $R^{3'}$ are each independently H, substituted or unsubstituted saturated or partially unsaturated hydrocarbyl as defined above under (i) for $R^1$ and $R^{1'}$; or

(iii)
$R^1$ and $R^{1'}$ are each independently H, substituted or unsubstituted saturated or partially unsaturated hydrocarbyl as defined above under (i) for $R^1$ and $R^{1'}$; and
-$R^2$ and $R^3$ together with the carbon atom ($C^1$) to which they are attached form an unsubstituted or substituted saturated or partially unsaturated carbocyclic ring moiety of 3 to 14 C-atoms, preferably 5-12 C atoms; or an unsubstituted or substituted saturated or partially unsaturated heteroring moiety of 3 to 14 ring atoms comprising 1 to 6, preferably 1 to 4 heteroatoms, selected from O, N, P, S or Si;

- wherein said carbocyclic ring or heteroring is optionally fused with another optionally substituted ring system having 4 to 14 ring atoms; and

- wherein said substituted carbocyclic ring or heteroring system comprises 1 to 4 substituents selected independently from a functional group, or a saturated or partially unsaturated hydrocarbyl optionally bearing a functional group; and

-$R^{2'}$ and $R^{3'}$ together with the carbon atom ($C^{1'}$) to which they are attached form an unsubstituted or substituted saturated or partially unsaturated carbocyclic ring moiety of 3 to 14 C-atoms, preferably of 5-12 C atoms; an unsubstituted or substituted saturated or partially unsaturated heteroring moiety of 3 to 14 ring atoms comprising 1 to 6, preferably 1 to 4 heteroatoms, selected from O, N, P, S or Si;

- wherein said carbocyclic ring or heteroring system is optionally fused with another optionally substituted ring system having 4 to 14 ring atoms; and

- wherein said substituted carbocyclic ring or heteroring system comprises 1 to 4 substituents selected independently from a functional group or a saturated or partially unsaturated hydrocarbyl optionally bearing a functional group;

- with a proviso for alternatives (i) to (iii) that at least two of $R^1$, $R^2$ and $R^3$, and at least two of $R^{1'}$, $R^{2'}$ and $R^{3'}$, respectively, are other than H or methyl.

The compounds of formula (V) are preferably selected from the alternatives (ii) or (iii), more preferably from alternative (iii).

The substituents $R^1$, $R^2$, $R^3$, $R^{1'}$, $R^{2'}$ and $R^{3'}$ of compounds of formula (I) or (V) may each independently optionally carry 1 to 4 substituents as defined above. Said optional substituents may preferably be selected each independently from a functional group as defined above; saturated or partially unsaturated hydrocarbyl optionally bearing a functional group; or aromatic hydrocarbyl optionally bearing a functional group, as defined above, preferably from C1-12 hydrocarbyl (e.g. C1-6 alkyl) or from a functional groups as defined above. If a substituent is present, preferably 1 substituent is present.

Preferred aspects discussed above and below with respect to formula (I) can also apply to compounds of formula (V).

The following subgroups of the compound of formula (I) of the invention represent some preferable embodiments and variants of the invention. It is also understood that said below subgroups further specify the substituents given above in formula (I). Each subgroups definition can be combined with any other subgroup to define further preferred subgroups within the broadest scope of compounds of formula (I) of the invention.

Moreover said above generally defined compounds of first, second and third group and said subgroups thereof, and the general definition for compounds of formula (I), as well as said subgroups thereof, can be combined in any combination in their uses for modifying polymers, to modification methods, to modified polymers and to articles comprising said modified polymers, as well as to preparation process thereof, which all aspects of the invention are discussed below..

In a preferred embodiment of the invention compounds of formula (I) are symmetrical.

A first preferable embodiment (A) comprises a subgroup (1) of the compound of formula (1) as defined above, wherein $R^2$ and $R^3$ together with carbon atom ($C^1$) to which they are attached form an optionally substituted carbocyclic ring moiety of 3 to 12 ring C-atoms or an optionally substituted heteroring moiety of 3 to 12 ring atoms containing 1 to 6, preferably 1 to 4, heteroatoms selected from O, N, P, S or Si, and wherein said carbocyclic or heterocyclic ring system is optionally fused with another ring system having 4 to 14 ring atoms. This optionally fused ring system may also carry substituents, e.g. 1 to 4 groups Z as herein defined or may be unsubstituted.

Preferably $R^2$ and $R^3$ together with carbon atom ($C^1$) form a (C3-C12) carbocyclic ring moiety. The (C3-C12) carbocyclic ring moiety may optionally be substituted with 1 to 4 substituents which are preferably selected from substituents (Z) as defined later below.

In a subgroup (2) of the compound of formula (I) as defined above, $R^{2'}$ and $R^{3'}$ together with carbon atom (C1') to which they are attached form an optionally substituted carbocyclic ring moiety of 3 to 12 ring C-atoms or an optionally substituted heteroring moiety of 3 to 12 ring atoms containing 1 to 6, preferably 1 to 4, heteroatoms selected from O, N, P, S or Si, and wherein said carbocyclic or heterocyclic ring system is optionally fused with another ring system having 4 to 14 ring atoms. This optionally fused ring system may also carry substituents e.g. 1 to 4 groups Z as herein defined or may be unsubstituted

Preferably $R^{2'}$ and $R^{3'}$ together with carbon atom ($C^{1'}$) form a (C3-C12) carbocyclic ring moiety. Said (C3-C12) carbocyclic ring moiety may optionally be substituted with 1 to 4 substituents which are preferably selected from substituents (Z) as defined later below.

In a subgroup (3) of the compound of formula (I) as defined above, $R^2$ and $R^3$ together with the carbon atom ($C^1$) to which they are attached form an optionally substituted, saturated or partially unsaturated mono- or bicyclic (C4-C14) carbocyclic ring, preferably unsubstituted saturated monocyclic (C5-C8) carbocyclic ring, such as cyclopentyl, cyclohexyl or cycloheptyl, preferably cyclohexyl or cyclopentyl. Also preferably, in said subgroup (3), $R^2$ and $R^3$ together with the carbon atom ($C^1$) to which they are attached form a saturated monocyclic (C5-C8) carbocyclic ring, such as cyclopentyl, cyclohexyl or cylcoheptyl, preferably cyclohexyl or cyclopentyl, which is substituted with 1 to 4 substituents which are preferably selected from substituents (Z) as defined later below.

In a subgroup (4) of the compound of formula (I) as defined above, $R^{2'}$ and $R^{3'}$ together with the carbon atom ($C^{1'}$) to which they are attached form an optionally substituted, saturated or partially unsaturated mono- or bicyclic (C4-C14) carbocyclic ring, preferably unsubstituted saturated monocyclic (C5-C8) carbocyclic ring, such as cyclopentyl, cyclohexyl or cylcoheptyl, preferably cyclohexyl or cyclopentyl. Also preferably in said subgroup (4) $R^{2'}$ and $R^{3'}$ together with the carbon atom (C1') they are attached to form a saturated monocyclic (C5-C8) carbocyclic ring, such as cyclopentyl, cyclohexyl or cylcoheptyl, preferably cyclohexyl or cyclopentyl, which is substituted with 1 to 4 substituents which are preferably selected from substituents (Z) as defined later below, e.g. one substituent Z.

More preferably, in a subgroup (5a) of the compounds (I), $R^2$ and $R^3$ and, respectively, $R^{2'}$ and $R^{3'}$ form carbocyclic rings as defined in formula (I), more preferably form carbocyclic rings as defined in subgroups (1) and, respectively, (2), even more preferably form carbocyclic rings as defined in subgroups (3) and, respectively (4), which may be substituted with 1 to 4 substituents which are preferably selected from substituents (Z) as defined later below, e.g. one substituent Z.

In an even preferable subgroup (5b) of the compound of formula (I) as defined above, $R^2$ and $R^3$ together with the carbon atom ($C^1$) to which they are attached form a ring system as defined in subgroup (3) and $R^{2'}$ and $R^{3'}$ together with the carbon atom ($C^{1'}$) to which they are attached form a ring system as defined in subgroup (4), whereby the ring system formed by $R^{2'}$ and $R^{3'}$ together with the carbon atom ($C^{1'}$) is identical to the ring system formed by $R^2$ and $R^3$ together with the carbon atom ($C^1$).

Subgroups 1 to 5b form part of embodiment (A) of the invention, i.e. where the substituents $R^1$ and $R^{1'}$ are as defined in formula (I) above. These subgroups can be combined with any $R^1$ and $R^{10}$ substituent.

Highly preferred subgroups of embodiment (A), are the subgroup (5a) and even more preferably subgroup (5b).

A second preferable embodiment (B) comprises a subgroup (6) of the compound of formula (I) as defined above, wherein $R^1$, $R^2$, $R^3$, $R^{1'}$, $R^{2'}$ and $R^{3'}$ each independently is optionally substituted mono- or multicyclic (C5-C14) aryl; optionally substituted mono- or multicyclic (C5-C14)heteroaryl; optionally substituted mono- or multicyclic (C4-C14) cycloalkyl; optionally substituted mono- or multicyclic (C4-C14)heterocyclyl; optionally substituted straight or branched chain (C1-C50)alkyl, preferably straight chain (C1-C30)alkyl; optionally substituted straight or branched chain, preferably straight chain, (C2-C50)alkenyl, preferably straight chain (C2-C30)alkenyl; or optionally substituted straight or branched chain, preferably straight chain, (C2-C50)alkynyl, preferably straight chain (C2-C30)alkynyl; or optionally substituted straight or branched chain (C1-C50)heteroalkyl comprising 1 to 4 heteroatoms selected from N, O, S or Si. The optionally substituted moieties as defined above contain preferably 1 to 4 substituents which are preferably selected from substituents (Z) as defined later below.

Preferable embodiments (B) of compounds (I) are any of subgroups (7) to (11), optionally in any combinations thereof:.

In a subgroup (7) of the compound of formula (I) as defined above, $R^2$, $R^{2'}$, $R^3$ and $R^{3'}$ are each independently selected from unsubstituted straight chain (C1-C50)alkyl, preferably (C1-C30)alkyl, more preferably (C1-C20)alkyl, such as hexyl, heptyl, octyl, decyl, undecyl, docedyl, preferably decyl.

In a subgroup (8) of the compound of formula (I) as defined above, $R^2$ and $R^{2'}$ each represents same radical and, respectively, $R^3$ and $R^{3'}$ each represents same radical.

In a subgroup (9) of the compound of formula (I) as defined above, $R^2$ and $R^{2'}$ are same and each represents methyl.

In a subgroup (10) of the compound of formula (I) as defined above, $R^2$ and $R^{2'}$ are same and each represents (C6-C30)alkyl.

In a subgroup (11) of the compound of formula (I) as defined above, $R^3$ and $R^{3'}$ are same and each represents (C6-C30)alkyl.

A third preferable embodiment (C) of the compounds (I) is a subgroup (12). In a subgroup (12) of the compound of formula (I) as defined above $R^1$ and $R^{1'}$ are same or different, preferably same, and each represents optionally substituted, saturated or partially unsaturated cyclic hydrocarbyl of 5 to 14 ring atoms optionally containing 1 to 4 heteroring atoms selected from N, O, P, S or Si, or optionally substituted mono- or multicyclic (C5-C14)aryl, preferably unsubstituted monocyclic (C5-C7)aryl. Also preferably in said subgroup (12) $R^1$ and $R^{1'}$ are same or different, preferably same and each represents substituted mono- or multicyclic (C5-C14)aryl, preferably monocyclic (C5-C7)aryl which is substituted with 1 to 4 substituents which are preferably selected from substituents (Z) as defined later below.

A fourth preferable embodiment (D) of the compounds (I) is a subgroup (13). In a subgroup (13) of the compound of formula (I) as defined above, $R^1$ and $R^{1'}$ are same or different, preferably same, and each represents optionally substituted branched or straight chain, preferably unsubstituted straight chain, (C6-C30)alkyl or methyl. This embodiment also covers the option that $R^1$ and $R^{1'}$ are same or different, preferably same, and each represents optionally substituted branched or straight chain, preferably unsubstituted straight chain, (C2-C5)alkyl.

For the avoidance of doubt it is stressed that the preferred definitions of $R^1$ and $R^{1'}$ given in subgroups 12 and 13 can be combined with any of the preferred substituent definitions of subgroups 1 to 11 to form even more preferred compounds.

Further preferred compounds of formula (I) are of subgroup (14) with the further proviso that at least two of $R^1$, $R^2$ and $R^3$, and at least two of $R^{1'}$, $R^{2'}$ and $R^{3'}$, respectively, are other than H, methyl, iso-butyl or tert-butyl.

Further preferred compounds of formula (I) are of subgroup (15) with the further proviso that at least two of $R^1$, $R^2$ and $R^3$, and at least two of $R^{1'}$, $R^{2'}$ and $R^{3'}$, respectively, are other than H, methyl, ethyl, 1-propyl, isopropyl, 1-butyl, iso-butyl or tert-butyl.

Further preferred compounds of formula (I) are of subgroup (16) with the further proviso that at least two of $R^1$, $R^2$ and $R^3$, and at least two of $R^{1'}$, $R^{2'}$ and $R^{3'}$, respectively,, are each other than $CH_3$ preferably other than straight or branched chain saturated or partially unsaturated (C1-C3)hydrocarbyl, more preferably other than straight or branched chain saturated or partially unsaturated (C1-C4)hydrocarbyl,

Further preferred compounds of formula (I) are of subgroup (17) with the further proviso that at least two of $R^1$, $R^2$ and $R^3$, and at least two of $R^{1'}$, $R^{2'}$ and $R^{3'}$, respectively, are preferably other than straight or branched chain saturated or partially unsaturated (C2-C3)hydrocarbyl, more preferably other than straight or branched chain saturated or partially unsaturated (C2-C4)hydrocarbyl.

Each of subgroups (14), (15) , (16) and (17) are useful for embodiments wherein very high purity products, e.g. polymers, are desirable after the modification step with compound (I).

Further preferable compounds of formula (I) as defined above form subgroup (Ia). In this subgroup $R^1$ and $R^{1'}$ are same or different, preferably same, and each represents optionally substituted branched or straight chain, preferably unsubstituted straight chain, (C2-C30)alkyl, which is preferably (C6-C30)alkyl; or methyl, more preferably methyl; and

$R^2$ and $R^3$ together with $C^1$ atom to which they are attached form an optionally substituted, saturated or partially unsaturated mono- or bicyclic (C4-C14)carbocyclic ring, preferably optionally substituted, more preferably unsubstituted saturated monocyclic (C5-C8)carbocyclic ring;
and $R^{2'}$ and $R^{3'}$ together with the carbon atom ($C^{1'}$) to which they are attached form an optionally substituted, saturated or partially unsaturated mono- or bicyclic (C4-C14)carbocyclic ring, preferably optionally substituted, more preferably unsubstituted saturated monocyclic (C5-C8)carbocyclic ring; whereby the ring system formed by $R^2$ and $R^3$ together with $C^1$ is preferably identical to a ring system formed by $R^{2'}$ and $R^{3'}$ together with $C^{1'}$.

Any substituted moiety preferably contains 1 to 4 substituents (Z) as defined later below, e.g. one substituent Z.

Especially preferred cyclic radicals are cyclopentyl and cyclohexyl in this subgroup.

One of the preferred compounds of this more preferable subgroup of (Ia) is the compound of formula (Ia) which is di-(1-methylcyclohexyl) peroxide (formula Ia):

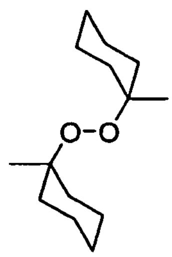

(Ia)

Another preferred compound is (Ia'), di(1-methylcyclopentyl) peroxide.

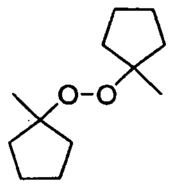

(Ia')

A second preferred subgroup (Ib) of compounds (I) is an embodiment (B) as defined above, wherein $R^2$, $R^{2'}$, $R^3$ and $R^{3'}$ are as defined in subgroup (6) above, preferably as defined in subgroups (7) to (11), and $R^1$ and $R^{1'}$ are according to preferable embodiment (C).

In preferable subgroup (Ib) of compounds of formula (I) as defined above, $R^1$ and $R^{1'}$ are both same and represent an optionally substituted, preferably unsubstituted, monocyclic (C5-C7)aryl;
$R^2$ and $R^{2'}$ are same and are both methyl; and
$R^3$ and $R^{3'}$ are same and are both optionally substituted branched or straight chain (C6-C50)alkyl, more preferably unsubstituted straight chain (C6-C30)alkyl, such as (C6-C20)alkyl.

Any substituted moiety preferably contains 1 to 4 substituents (Z) as defined later below, e.g. one substituent Z.

One of the preferred compounds of formula (I) of this preferable subgroup of (Ib) the compound of formula (Ib) which is Di-(1-methyl-1-phenylundecyl) peroxide (formula Ib):

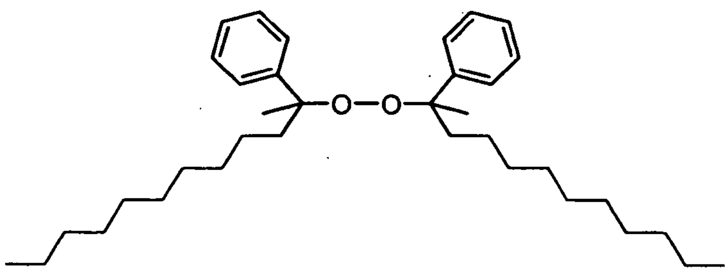

(Ib).

Other preferred compounds of subgroup (Ib) include di-(1-methyl-1-phenylheptyl) peroxide

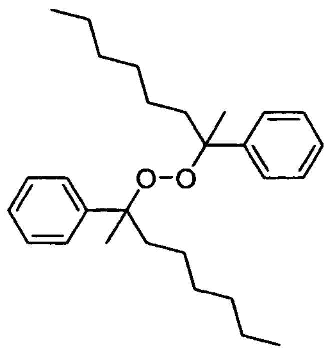

(Ib')

A third preferred subgroup (Ic) of compounds (I) is an embodiment (B) as defined above, wherein $R^2$, $R^{2'}$, $R^3$ and $R^{3'}$ are as defined in subgroups (7), (8), (10) or (11) and $R^1$ and $R^{1'}$ are according to preferable embodiment (C) or (D).

In one preferable subgroup (Ic) of compounds of formula (I) as defined above, $R^1$ and $R^{1'}$ are both same and represent an optionally substituted, preferably unsubstituted, monocyclic (C5-C7)aryl;
$R^2$ and $R^{2'}$ are same and are both optionally substituted branched or straight chain, preferably unsubstituted straight chain, (C6-C50)alkyl, more preferably unsubstituted straight chain (C6-C30)alkyl, such as (C6-C20)alkyl; and
$R^3$ and $R^{3'}$ are same and are both optionally substituted branched or straight chain, preferably unsubstituted straight chain, (C6-C50)alkyl, more preferably unsubstituted straight chain (C6-C30)alkyl, such as (C6-C20)alkyl.

In a further preferable subgroup (Id) of compounds (I), $R^1$ and $R^{1'}$ are according to embodiment (D), preferably $R^1$ and $R^{1'}$ are same and are both methyl; and $R^2$ and $R^{2'}$ are same and are both optionally substituted branched or straight chain, preferably unsubstituted straight chain, (C6-C50)alkyl, more preferably unsubstituted straight chain (C6-C30)alkyl, such as (C6-C20)alkyl; and $R^3$ and $R^{3'}$ are same and are both optionally substituted branched or straight chain, preferably unsubstituted straight chain, (C6-C50)alkyl, more preferably unsubstituted straight chain (C6-C30) alkyl, such as (C6-C20)alkyl.

In other preferred embodiments of the invention none of $R^1$-$R^3$ or $R^{1'}$-$R^{3'}$ represents an aromatic group.

Where one or more of $R^1$-$R^3$ or $R^{1'}$-$R^{3'}$ represents an aromatic radical this is especially preferably a phenyl group optionally substituted by one to three, such as one, group Z as hereinbefore defined.

Where one or more of $R^1$-$R^3$ or $R^{1'}$-$R^{3'}$ represents a cycloalkyl radical this is especially preferably a cyclohexyl or cyclopentyl group optionally substituted by one to three, such as one, group Z as hereinbefore defined.

In the compounds of formula (I) there are preferably no more than two cycloalkyl groups. In further preferred compounds there are no more than two cyclic groups (e.g. carbocyclic, heterocyclic or aromatic groups) in the compound of formula (I). In a most preferred embodiment of the invention there are two cyclic groups which each are formed by $R^2$ and $R^3$ together with $C^1$ and by $R^{2'}$ and $R^{3'}$ together with $C^{1'}$.

These preferred embodiments apply to any compound of formula (I), in particular any compounds forming part of the sub groups above.

The optional substituents of embodiments (A), (B), (C), (D), (Ia), (Ib), (Ic) and (Id) (and any optional substituent present in the compounds of the invention) are preferably one to four substituents (Z) selected from a saturated or partially unsaturated (C1-C30)hydrocarbyl, a functional group, a saturated or partially unsaturated (C1-C30)hydrocarbyl which optionally bears a functional group as defined above, or from an aromatic hydrocarbyl, which optionally bears a functional group. Preferred substiutents (Z) are branched or straight chain (C1-C20)hydrocarbyl or a functional group as defined above. Preferably, no substituted radical should carry more than 1 substituent Z.

Highly preferred substituents (Z) which can be present on any optionally substituted moiety of the compounds of the invention include C1-6 alkyls, especially methyl, ethyl, propyl or tertbutyl; C5-8 cycloalkyl; or phenyl. Where a methyl substituent carries a phenyl side group, the formed group is, of course, benzyl. Where an alkyl substituent carries a cycloalkyl side group, the formed group is, of course, alkylcycloalkyl, and so on.

Where a phenyl group carries one substituent it is preferably para to the binding to carbon arom $C^1/C^{1'}$. Where a cyclohexyl group carries one substituent, it is preferably beta to the $C^1/C^{1'}$ carbon atom.

The most preferred compounds of formula I are compounds according to subgroups (Ia), (Ia'), (Ib) and (Ib').

Said specific compounds of formula (Ia), (Ib), (Ia') and (Ib') are novel as such. The invention is further directed to the compound of formula (Ia) as defined above. Thus the invention is directed to the compound of formula (Ib) as defined above. The invention is further directed to the compound of formula (Ia') as defined above. The invention is further directed to the compound of formula (Ib') as defined above.

The most preferred subgroups of formula (I) and of formula (V) are subgroups Ia and Ib, even preferably subgroups (Ia) including the compounds (Ia) and (Ia').

Highly preferred compounds of the invention are therefore of formula (II)

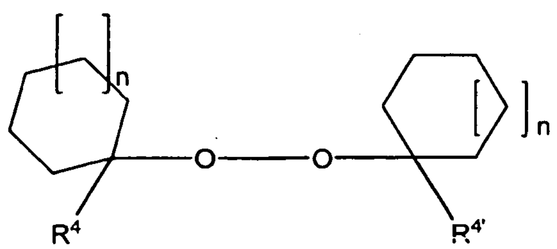

(II)

wherein n 0 to 3, preferably 0 or 1 forming a cyclopentyl or cyclohexyl group respectively, $R^4$ and $R^{4'}$ each independently represent a straight chain alkyl group having 1 to 30 carbon atoms, preferably methyl or straight chain alkyl group having 6 to 20, preferably 6 to 12, carbon atoms, more preferably methyl, and

wherein one or preferably both ring systems independently are unsubstituted or optionally substituted by I to 4 substituents Z as defined above. It is most preferred that the ring systems are unsubstituted..

Further highly preferred compounds of the invention are also of formula (III)

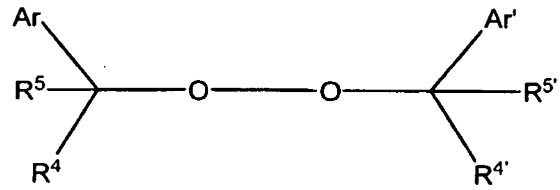

(III)

wherein Ar and Ar' independently represent a phenyl, benzyl or naphthyl group optionally substituted by 1 to 4 substituents Z as defined above,
$R^4$ and $R^{4'}$ each are methyl; and
$R^5$ and $R^{5'}$ each independently represent a straight chain alkyl group having C6-30 carbon atoms, preferably 6 to 20, more preferably 6 to 12 carbon atoms,

Most preferred is compounds of formula (II) as defined above.

The amount of the compound of formula (I) used as a free radical generating agent (B) in the Polymer Compostion is not critical and can vary depending on the desired crosslinking degree and the type of the crosslinkable polymer. As an example only, the amount of compound of formula (I) may be less than 10.0 wt%, less than 6.0 wt%, less than 5.0 wt%, less than 3.5 wt%, e.g. between 0.1 to 3.0 wt%, such as 0.2 to 2.6 wt%, based on the weight of the polymer composition. Factors affecting the amount of free radical generating agent (B) in the Polymer composition include the molecular weight of Compound of formula (I) and the desired degree of crosslinking.

**Preparation of the compounds of formula (I)**

The compounds of the invention include novel and known compounds. The use of the known compounds as a free radical generating agent, preferably for modifying a polymer composition, is novel. Thus said known compounds may be commercially available. Alternatively, the compounds of the invention can be prepared according to or analogously to known methods described in the chemical literature.

As an example, the compounds (I) as defined above can be prepared according to the following scheme 1 using known procedures which are described in a literature and well known for a skilled person in the art.

Peroxides of formula (I) as defined above can be prepared in several known ways, and more specifically tertiary peroxides can be prepared from the corresponding tertiary alcohols under acidic conditions to give compounds (I). The alcohols are either commercially available, or can be prepared from a suitable ketone combined with a organometallic reagent, more specifically a Grignard (RMgX, where X is an halogen) or organolithium (RLi) reagent.

**Scheme 1**

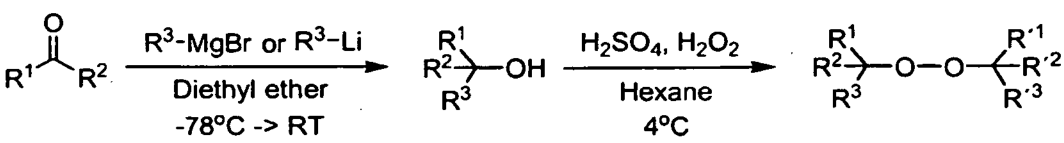


References to synthetic methods are as follows:

1) Milas, N.A., Surgenor, D.M., J. Am. Chem. Soc., 643-644, 1946
2) Hey, D.H., Stirling, C.J.M., Williams, G.H, J. Chem. Soc., 1054-1058, 1957
3) Organic Synthesis, Smith, M.B., The McGraw-Hill Companies Inc., 2002

Work up procedures are routine. The formed tertiary alcohol and corresponding peroxide can be purified by removing the solvent *in vacuo* and purifying the residue by any of the methods known to those skilled in the art, such

as crystallization.

Compounds of formula (I) can also be prepared from tertiary alcohols via conversion to a hydroperoxide -OOH type compound. This process allows the preparation of asymmetrical peroxides. Thus for example, a tertiary alcohol can be converted to a tertiary halide and reacted with hydrogen peroxide, perhaps in the presence of a promoter such as silver trifluoroacetate and a non nucleophilic base such as sodium hydrocarbonate to form a teriary hydroperoxide. The tertiary hydroperoxide can then be reacted with the a tertiary bromide (perhaps the same as used earlier in the reaction or optionally a difference tertiary bromide) to form the final diperoxide materials of formula (I). Again, a promoter such as silver trifluoroacetate/NaHCO3 might be used. These reactions are summarised in the scheme below:

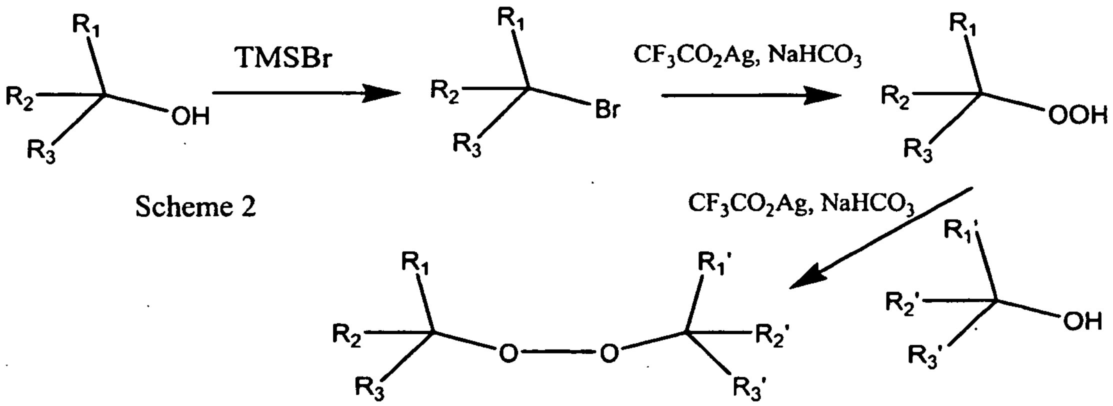


Scheme 2

In view of the low levels of volatile decomposition products formed during activation of the peroxides of the invention, the present invention reduces or minimises the fire, explosion and health risks in an working environment caused by the use of free radical generating agents compared to the prior art.

The first group and the second of compounds of the invention as defined above and in claim 1-3 in terms of the decomposition product(s) thereof, the third group of compounds of formula (I) of the invention as defined above with a general formula and by means of the preferable subgroups, in any combinations, as well as in claims 4-15 below, are abbreviated herein below as "Compound of the invention" for the sake of convenience, only. The preferred subgroup of Compound of the invention is compounds of formula (I) as defined above and in claims.

**The unsaturated polymer component (A) of Polymer Composition**

In a preferred embodiment of the Polymer Composition, the at least one unsaturated polymer (A) contains carbon-carbon double bonds in an amount of at least 0.05, e.g. 0.1 or more, more preferably of 0.2 or more, and most preferably more than 0.37 carbon-carbon double bonds/1000 carbon atoms, e.g. at least 0.40 carbon-carbon double bonds/1000 carbon atoms.

The at least one unsaturated polymer (A) can be a homopolymer, wherein the unsaturation is provided by a chain transfer agent, or a copolymer, wherein the unsaturation is provided by polymerizing a monomer in the presence of at least a polyunsaturated comonomer and optionally in the presence of a chain transfer agent.

The unsaturated polymer (A) preferably contains carbon-carbon double bonds in an amount of at least 0.6/1000 carbon atoms, or preferably at least 0.8/1000 carbon atoms. The upper limit of the amount of said carbon-carbon double bonds present in the unsaturated polymer (A) is not limited and may preferably be less than 5.0/1000 carbon atoms, preferably less than 3.0/1000 carbon atoms, more preferably of less than 2.5/1000 carbon atoms, especially less than 1.8/1000 carbon atoms.

Preferably, said carbon-carbon double bonds present in the unsaturated polymer (A) include vinyl groups, which vinyl groups originate preferably from i) a polyunsaturated comonomer, from ii) a chain transfer agent, or from iii) any mixture thereof.

More preferably, said C-C double bonds present in the unsaturated polymer include said vinyl groups in a total amount, in the given preference order, of at least 0.05 or more, e.g. 0.1/1000 carbon atoms, at least 0.2/1000 carbon atoms, 0.3/1000 carbon atoms, at least 0.4/1000 carbon atoms. In embodiments where higher vinyl content is desired the following ranges are preferable in the given preference order, at least 0.5/1000 carbon atoms, at least 0.6/1000 carbon atoms, or of at least 0.7/1000 carbon atoms. The upper limit of the total amount of said vinyl groups present in the unsaturated polymer (A) is typically as given above for C-C double bonds.

In one preferred embodiment the unsaturated polymer (A) is an unsaturated copolymer containing at least one

or more unsaturated comonomer(s). More preferably, said C-C double bonds present in the unsaturated copolymer include vinyl groups which originate from said one or more polyunsaturated comonomer(s). Preferably, the total amount of said vinyl groups which originate from the polyunsaturated comonomer is, in the given preference order, of at least 0.02/1000 carbon atoms, 0.05/1000 carbon atoms, 0.10/1000 carbon atoms. In embodiments where higher vinyl contents originating from polyunsaturated comonomer is desired then the following ranges in given preference order are preferred 0.15/1000 carbon atoms, 0.20/1000 carbon atoms, at least 0.25/1000 carbon atoms, at least 0.30/1000 carbon atoms, at least 0.35/1000 carbon atoms. The upper limit of the amount of said vinyl groups which originate from the polyunsaturated comonomer and contribute to the total amount of said C-C double bonds present in the unsaturated copolymer is not limited and may be, in the given preference order, of less than 3.0/1000 carbon atoms, less than 2.5/1000 carbon atoms, less than 2.0/1000 carbon atoms, less than 1.5/1000 carbon atoms.

When the unsaturated polymer (A) of the Polymer Composition, is an unsaturated copolymer, then the polyunsaturated comonomer(s) has/have preferably a straight carbon chain with at least 8 carbon atoms and at least 4 carbon atoms between the non-conjugated double bonds, of which at least one is terminal.

As to suitable unsaturated polymer materials for the Polymer Composition, said unsaturated polymer (A) can be any unsaturated polymer, preferably any unsaturated polymer having a double bond content as defined above for the unsaturated polymer (A) of the preferable Polymer Composition. The unsaturated polymer (A) is preferably a polyolefin which means a homopolymer of olefin or a copolymer of olefin with one or more comonomer(s). Said unsaturated polyolefin is preferably an unsaturated polyethylene or polypropylene. The unsaturated polyolefin can be unimodal or multimodal with respect to molecular weight distribution and/or comonomer distribution, which expressions have a well known meanings.

In the preferred embodiment of the Polymer Composition, said unsaturated polyolefin (A) is an unsaturated copolymer of olefin with at least one polyunsaturated comonomer and optionally with a further comonomer. As well known "Comonomer" refers to copolymerisable comonomer units.

The unsaturated copolymer of olefin is preferably an unsaturated copolymer of ethylene or an unsaturated copolymer of propylene.

Where the unsaturated copolymer of olefin is a polypropylene (PP) copolymer with at least one polyunsaturated comonomer and optionally with further comonomer(s), it can be a random copolymer of propylene or a heterophasic propylene copolymer, which have unsaturation in a manner known in the art. The unsaturated propylene copolymer is preferably produced by a conventional low pressure polymerization which is well documented and described in the polymer literature.

In the most preferable embodiment the unsaturated copolymer is a copolymer of ethylene.

The copolymer of ethylene may be a low density polyethylene (LDPE) copolymer produced in a high pressure polymerisation process, wherein ethylene is copolymerised with at least one polyunsaturated comonomer and optionally with a further comonomer(s), optionally in the presence of a chain transfer agent; or it may be a linear low density polyethylene (LLDPE) produced in a low pressure process, wherein ethylene is copolymerised at least with a polyunsaturated comonomer and optionally with a further comonomer in the presence of a coordination catalyst, such as chromium, Ziegler-Natta or single site catalyst. Both LDPE copolymers and LLDPE copolymers and the polymerisation processes thereof are well known.

The optional further comonomer(s) present in the unsaturated copolymer, preferably copolymer of ethylene, is different from the "backbone" monomer and may be selected from an ethylene and higher alpha-olefin(s), preferably $C_3$-$C_{20}$alpha-olefin(s), such as propylene, 1-butene, 1-hexene, 1-nonene or 1-octene, as well as from polar comonomer (s). It will be appreciated that where the main monomer is ethylene then the comonomer must be other than ethylene and where the main monomer is propylene then the comonomer is other than propylene, e.g. ethylene or $C_4$-$C_{20}$alpha-olefin

It is well known that, for example, propylene can be used as a comonomer or as a chain transfer agent (CTA), or both, whereby it can contribute to the total amount of the C-C double bonds, preferably to the total amount of the vinyl groups. Herein, when a copolymerisable CTA, such as propylene, is used, the copolymerised CTA is not calculated to the comonomer content.

In a preferred embodiment of the Polymer Composition, the unsaturated polymer (A) is an unsaturated LDPE copolymer containing at least one comonomer which is a polyunsaturated comonomer (referred below as LDPE copolymer).

More preferably, said polyunsaturated comonomer is a diene, preferably a diene which comprises at least eight carbon atoms, the first carbon-carbon double bond being terminal and the second carbon-carbon double bond being non-conjugated to the first one (group 1 dienes). Preferred dienes are selected from $C_8$ to $C_{14}$ non-conjugated dienes or mixtures thereof, more preferably selected from 1,7-octadiene, 1,9-decadiene, 1,11-dodecadiene, 1,13-tetradecadiene, 7-methyl-1,6-octadiene, 9-methyl-1,8-decadiene, or mixtures thereof. Even more preferably, the diene is selected from 1,7-octadiene, 1,9-decadiene, 1,11-dodecadiene, 1,13-tetradecadiene, or any mixture thereof.

In addition or as an alternative to the dienes listed above, the diene may also be selected from one or more

siloxane compounds having the following formula (group 2 dienes):

$$CH_2=CH-[SiR_xRy-O]_z\ SiR_xR_y-CH=CH_2,$$

wherein z = 1 to 200, and
$R_x$ and $R_y$, which can be the same or different, are selected from $C_1$, to $C_4$ alkyl groups and/or $C_1$ to $C_4$ alkoxy groups.

Preferably, $R_x$ and/or $R_y$ are methyl, methoxy or ethoxy. Preferably, z = 1 to 100, more preferably 1 to 50. As an example, divinylsiloxanes such as $\alpha,\omega$-divinylsiloxane can be mentioned.

In addition or as an alternative to the group 1 and 2 dienes listed above, the diene may also be selected from one or more ether compounds having the following formula:

$$CH_2=CH-O-R_q-CH=CH_2$$

wherein
Rq is $-(CH_2)_m-O-$, $-(CH_2CH_2O)_p-$, or $-CH_2-C_6H_{10}-CH_2-O-$, m is 2 to 10, and p is 1 to 5 (group 3 dienes).

Preferred polyunsaturated comonomers for said unsaturated copolymer are the dienes from group 1 as defined above. It is also preferred that said unsaturated copolymer is the above-mentioned unsaturated LDPE copolymer. It may contain further comonomers, e.g. polar comonomer(s), alpha-olefin comonomer(s), or any mixture thereof.

As a polar comonomer, compound(s) containing hydroxyl group(s), alkoxy group(s), carbonyl group(s), carboxyl group(s), ether group(s) or ester group(s), or a mixture thereof can used. More preferably, compounds containing carboxyl and/or ester group(s) are used and still more preferably, the compound is selected from the groups of acrylate(s), methacrylate(s) or acetate(s), or any mixtures thereof.

If present in the unsaturated LDPE copolymer, the polar comonomer is preferably selected from the group of alkyl acrylates, alkyl methacrylates or vinyl acetate, or a mixture thereof. Further preferably, said polar comonomers are selected from $C_1$-to $C_6$-alkyl acrylates, $C_1$- to $C_6$-alkyl methacrylates or vinyl acetate. Still more preferably, said polar copolymer comprises a copolymer of ethylene with $C_1$- to $C_4$-alkyl acrylate, such as methyl, ethyl, propyl or butyl acrylate, or vinyl acetate, or any mixture thereof.

The unsaturated LDPE, preferably unsaturated LDPE copolymer, of the Polymer Composition is preferably produced at high pressure by radical polymerisation. High pressure polymerisation can be effected in a tubular reactor or an autoclave reactor. Preferably, it is a tubular reactor. When preparing the unsaturated LDPE copolymer in a high pressure process, polymerization is generally performed at a pressure of 100 to 400 MPa and a temperature of 150 to 350°C. The MFR can be adjusted using, for example, a chain transfer agent during the polymerisation, or by adjusting reaction temperature or pressure. The unsaturation, preferably C-C double bond content, can be adjusted by the polymerisation conditions, by polymerising the ethylene together with, for example, a polyunsaturated comonomer, chain transfer agent, or both. The feed ratio between C2 and polyunsaturated comonomer and/or chain transfer agent can be used to manipulate the amount of C-C double bonds present in the unsaturated LDPE copolymer. Preferably, at least the polyunsaturated comonomer is used for providing the unsaturation, preferably C-C double bonds. The high pressure polymerisation and the adjustment of process conditions to provide a desired MFR and unsaturation, preferably C-C double bond content, are known and described in the literature.

The unsaturated polymer (A) of the Polymer Composition of invention can be prepared using i.a. any conventional polymerisation process and equipment, the conventional means as described above for providing unsaturation in order to control and adjust the process conditions to achieve the desired unsaturation, preferably C-C double bond content, of the polymerised polymer. The double bond content can be further tailored depending on the desired embodiment. The unsaturated LDPE polymer as defined above, preferably the unsaturated LDPE copolymer, of the Polymer Composition is preferably produced in high pressure by free radical initiated polymerisation (referred to as high pressure radical polymerization). The usable high pressure polymerisation (HP) and the adjustment of process conditions are well known and described in the literature, and can readily be used by a skilled person to provide the above inventive balance. High pressure polymerisation can be effected in a tubular reactor or an autoclave reactor, preferably in a tubular reactor. One preferable HP process is described below for polymerising ethylene optionally together with one or more comonomer(s), preferably at least with one or more polyunsaturated comonomer(s), in a tubular reactor to obtain a LDPE homopolymer or copolymer as defined above. The process can be adapted to other polymers as well:

Compression:

Ethylene is fed to a compressor mainly to enable handling of high amounts of ethylene at controlled temperature. The compressors are usually a piston compressor or diaphragm compressors. The compressor is usually a series of compressors that can work in series or in parallel. Most common is 2-5 compression steps. Recycled ethylene and comonomers can be added at feasible points depending on the pressure. Temperature is typically low, usually in the

range of less than 200°C or less than 100°C.

Tubular reactor:

The mixture is fed to the tube reactor. First part of the tube is to adjust the temperature of the feed ethylene; usual temperature is 150-170°C. Then the radical initiator is added. As the radical initiator, any compound or a mixture thereof that decomposes to radicals at an elevated temperature can be used. Usable radical initiators are commercially available. The polymerization reaction is exothermic.

There can be several radical initiator injections points, e.g. 1-5 points or more, usually provided with separate injection pumps. There can also be additional monomer and/or comonomer injection points, e.g. 1-5 points or more, with separate compressors. The reactor is continuously cooled e.g. by water or steam. The highest temperature is called peak temperature and the lowest temperature is called radical initiator temperature.

Suitable temperatures range from 80 to 350°C and pressure from 100 to 400 MPa. Pressure can be measured at least in compression stage and after the tube. Temperature can measured at several points during all steps. Using various temperature profiles selected by a person skilled in the art will allow control of structure of polymer chain, i.e. Long Chain Branching and Short Chain branching, density, branching factor, distribution of comonomers, MFR, viscosity, Molecular Weight Distribution etc. E.g. the MFR of the unsaturated LDPE polymer (A), preferably unsaturated LDPE copolymer, can be adjusted by using e.g. chain transfer agent during the polymerisation, or by adjusting reaction temperature or pressure. The reactor ends conventionally with a valve. The valve regulates reactor pressure and depressurizes the reaction mixture from reaction pressure to separation pressure.

Separation:

The pressure is typically reduced to approx 35 MPa (40 MPa) bars or below. The polymer is separated gaseous products such as unreacted monomer and unreacted optional comonomer(s) and most of the unreacted gaseous products are recovered. Normally low molecular compounds, i.e. wax, are removed from the gas. The pressure can further be lowered to recover and recycle the unused gaseous products such as ethylene. The gas is usually cooled and cleaned before recycling.

Then the obtained polymer melt is normally pressurized mixed and pelletized. Preferably additives can be added in the mixer. Further details of the production of ethylene (co)polymers by high pressure radical polymerization can be found in in WO 93/08222, WO 9635732 and the Encyclopedia of Polymer Science and Engineering, Vol. 6 (1986), pp 383-410.

When the unsaturated LDPE copolymer of the invention is prepared, then, as well known, the unsaturation, preferably C-C double bond content, can be adjusted by polymerising the ethylene e.g. in the presence of one or more polyunsaturated comonomer(s), chain transfer agent(s), or both, using the desired feed ratio between C2 and polyunsaturated comonomer and/or chain transfer agent, depending on the nature and amount of C-C double bonds desired for the unsaturated LDPE copolymer. I.a. WO 9308222 describes a high pressure radical polymerisation of ethylene with polyunsaturated monomers, such as an $\alpha,\omega$- alkadienes, to increase the unsaturation of an ethylene copolymer. The non-reacted double bond(s) thus provides pendant vinyl groups to the formed polymer chain at the site, where the polyunsaturated comonomer was incorporated by polymerization. As a result the unsaturation can be uniformly distributed along the polymer chain in random copolymerisation manner. Also e.g. WO 9635732 describes high pressure radical polymerisation of ethylene and a certain type of polyunsaturated $\alpha,\omega$-divinylsiloxanes. Moreover, as known, propylene can be used as a chain transfer agent to provide said double bonds, whereby it can also partly be copolymerised with ethylene.

The alternative unsaturated LDPE homopolymer may be produced analogously using process conditions as for the unsaturated LDPE copolymer, except that ethylene is polymerised in the presence of a chain transfer agent only.

In a further preferable embodiment the $MFR_2$ of the unsaturated polymer (A), preferably an LDPE copolymer, is preferably from 0.01 to 50 g/10min, more preferably is from 0.1 to 20 g/10min, and most preferably is from 0.2 to 10 g/10min.

A preferable unsaturated polymer (A), preferably an LDPE copolymer, of the present invention may have a density, in the given order of preference, of higher than 0.860, higher than 0.880, higher than 0.900, higher than 0.910, or of higher than 0.915, $g/cm^3$,

A further preferable unsaturated polymer (A), preferably a LDPE copolymer, of the present invention may have a density of up to 0.960 $g/cm^3$, e.g. less than 0.955, e.g. less than 0.950, e.g. less than 0.945, e.g. less than 0.940, e.g. less than 0.935, or of less than 0.930, $g/cm^3$. The most preferred range is from 0.915 to 0.930 $g/cm^3$.

Further preferably, the unsaturated copolymer, preferably the LDPE copolymer, of the Polymer Composition may contain comonomer(s) in a total amount of up to 30 wt%, e.g. of from 0.05 to 25 wt.-%, or more preferably from 0.1 to 15 wt.-%, based on the amount of the unsaturated polymer component (A).

## End uses and end applications of the invention

### I. Crosslinking of polymers

The unsaturated polymers (A) of the Polymer Composition are crosslinkable.

The invention is further directed to the use of compounds of formula (I) of the invention as a free radical generating agent (B) for modifying, i.e. at least crosslinking the unsaturated polymers (A) by radical formation. Also a process for modifying the unsaturated polymer (A) by radical reaction using a free radical generating agent is provided, wherein said free radical generating agent is a compound of formula (I).

Accordingly, the modification of the polymer compostion comprises crosslinking the unsaturated polymer (A) by radical reaction using one or more free radical generating agents, wherein at least one said free radical generating agent is a compound of formula (I)_as defined above.

The term "crosslinking" is well known and commonly used in the polymer field and means forming, primarily, of interpolymer crosslinks (bridges) via radical reaction.

The amount of the compound of formula (I) used as a free radical generating agent for crosslinking is preferably as defined above in the description of Compound of formula (I) as a free radical generating agent (B).

The crosslinking may be carried out in a known manner, typically in elevated temperature, such as 140°C or more, preferably 150°C or more. And said step may be effected under atmospheric or typically slightly pressurised conditions, e.g. up to 20 bar, e.g. up to about 13 bar, pressure.

### III. End Applications

### 1. Article

The new principle of the invention is highly feasible in wide variety of end applications of polymers.

Accordingly, the invention further provides an article comprising the polymer composition of the invention as defined above and below, preferably under above "II. Polymer composition" or in claims, which is referred herein below as "polymer composition of the invention".

### 1.1 Cable

In one preferable embodiment said article of the invention is a cable comprising a conductor surrounded with one or more layers, wherein at least one layer comprises said polymer composition of the invention or a modified polymer composition of the invention.

The term "conductor" means herein above and below that the conductor comprises one or more wires. Moreover, the cable may comprise one or more such conductors. Preferably the conductor is an electrical conductor.

In one embodiment of the cable of the invention at least one layer is an insulation layer which comprises said polymer composition of the invention or a modified polymer composition of the invention.

In another embodiment of the cable of the invention at least one layer is a semiconductive layer comprising said polymer composition of the invention or a modified polymer composition of the invention. "Semiconductive layer" means herein that said layer comprises carbon black and has a volume resistivity of 100 000 Ω-cm or below when measured at 23°C or 90°C, or, when measured according to ISO 3915 using a plaque, has a volume resistivity of 100 Ω-cm or below at 23°C, or of 1000 Ω-cm or below at 90°C.

In further embodiment, the cable of the invention comprises a jacketing layer and optionally one or more layers selected from an insulation layer and semiconductive layer surrounded by said jacketing layer, wherein said jacketing layer comprises said polymer composition of the invention or a modified polymer composition of the invention.

As one further embodiment of the cable of the invention, a low voltage cable is provided which comprises an insulation layer and optionally a jacketing layer, wherein said insulation layer comprises said polymer composition of the invention.

As a further embodiment of the cable of the invention, a power cable is provided which comprises at least an inner semiconductive layer, insulation layer and an outer semiconductive layer, in that order, optionally surrounded by a jacketing layer, wherein at least one of said layers, preferably at least inner semiconductive layer and insulation layer, comprises said polymer composition of the invention.

In the context of the present invention, a low voltage cable is a cable operating in voltages 1 kV or below. A power cable is defined to be a cable transferring energy operating at any voltage, typically operating at voltages higher than 1 kV. The voltage applied to the power cable can be alternating (AC), direct (DC), or transient (impulse). In a preferred embodiment, the power cable prepared according to the present invention is operating at voltages higher than 6 kV and are known i.a. as medium voltage (MV), high voltage (HV) and extra high voltage (EHV) power cables, which

terms have well known meaning and indicate the operating level of such cable.

Said outer semiconductive layer of said power cable of the invention can be non-strippable, i.e. bonded and non-peelable, or strippable, i.e. non-bonded and peelable. Said terms have well known meanings in the wire and cable field.

## 2 Preparation process of an article

The present invention further provides a process for producing an article by using said polymer composition of the invention.

### 2.2. Preparation process of a cable

A preferable embodiment of the process for preparing an article of the invention is a process for producing a cable comprising steps of applying, preferably by (co)extrusion, one or more layers on a conductor, which layers comprise a polymer composition, wherein at least one layer comprises said polymer composition of the invention.

The term "(co)extrusion" means herein that in case of two or more layers, said layers can be extruded in separate steps, or at least two or all of said layers can be coextruded in a same extrusion step, as well known in the art.

In said process of the invention the components of a layer material are mixed in a separate mixer before introducing to the extruder for producing said layers or are added directly to an extruder and mixed therein before forming to a layer. Additives and further components can be added during the mixing step. The mixture in extruder is subjected to an elevated temperature, typically above the melting point of the polymer components and then (co)extruded on a conductor in a manner very well known in the field. E.g. conventional extruders and mixers may be used in the process of the invention.

A polymer powder, polymer pellets or melt, which comprise said polymer composition of the invention, can each equally be used in said process for preparing cables and they can be prepared prior their use in the cable preparation step or they can be prepared directly in a cable production line during a cable manufacturing process. Accordingly, 1) preformed powder or pellets of a polymer composition of the invention may be subjected to the cable production line; or 2) said compound of formula (I) may be added together with pellets or powder of the unsaturated polymer in a mixing step before forming the cable layer(s). Such mixing step can be a separate step in a separate mixing device arranged in the cable production line to precede the cable layer formation step, e.g. an extrusion step. Alternatively, the compound of formula (I) can be added during the layer formation step e.g. in an extruder, whereby it can be introduced to the extruder together with or after the addition of polymer powder or polymer pellets. The addition point in an extruder is not limited, whereby the compound of formula (I) can be added at the inlet of the extruder or at a later feed point arranged along the extruder. Accordingly, the addition of compound of formula (I) may take place at the time the polymer material is in solid non-molten, partly molten or molten state, i.e. a melt mixture. The obtained molten mixture of a layer material is then (co)extruded on to a conductor to form a cable layer. In a preferred cable preparation process of the invention a low voltage cable or, more preferably, a power cable of the invention as defined above under 1.1, cable is produced. The obtained cable can be further processed for the end use application.

Typically the cable of the invention is crosslinked after the formation of cable layers. The invention further provides a process (C1) for crosslinking a cable by radical reaction using a compound of formula (I), comprising step of:

(i) applying at least one layer comprising a polymer composition of the invention on a conductor,
(ii) crosslinking by radical reaction said at least one layer; and
(iii) recovering the crosslinked cable in a conventional manner for further use;

Preferably said crosslinking is effected producing methane as a decomposition product of said crosslinking step in an amount of less than 300 ppm (weight), when determined according to a method described below under "GC-analysis protocol". Preferably said crosslinking step (C1) is carried out without producing methane as a decomposition product of said crosslinking step.

In above crosslinking process of the invention crosslinking conditions can vary depending i.a. on the used materials and cable size. The crosslinking of the invention is effected e.g. in a known manner preferably in an elevated temperature. Preferably the lowest temperature in a cable layer during the crosslinking step is above 140°C, more preferably above 150°C, such as 160-210°C. The crosslinking may be carried out in a liquid or gas medium, such as in an inert gas, such as $N_2$, atmosphere. The pressure during the crosslinking step of the invention is typically up to 20 bar, preferably up to 13 bar, such as 10-15 bar, in inert atmosphere.

A further preferable embodiment of the crosslinking process of the invention comprises a further step of cooling the crosslinked cable preferably under pressurized conditions in a cooling medium e.g. in gas or liquid, such as $N_2$, oil or water. The cooling is effected in a cooling zone, which may be optionally integrated with the preceding crosslinking

zone, e.g. in a known vulcanization tube. As an example only, continuous catenary vulcanization (CCV) tube can be mentioned. The temperature at the layer closest to conductor is typically below 200°C, e.g. 160-190°C, at the beginning of the cooling zone/step. The pressure during the cooling step of the invention is typically kept above atmospheric pressure, e.g. up to 20 bar, preferably up to 13 bar, such as 10-12 bar. The cable is removed from the pressurized cooling step, when the temperature of the cable layers is clearly below the melting point of the polymer layer material thereof. Accordingly, the crosslinked cable of the invention may leave the pressurized cooling step of the invention e.g. when the temperature of the conductor of said cable is below 110°C depending on the layer polymer material, preferably between 70-90°C, at the exit of the pressurized cooling zone.

The crosslinking and cooling step is normally carried out under pressurized conditions to prevent the formation of voids due to volatile decomposition products of e.g. peroxides. In a preferable embodiment of the crosslinking process of the invention thus enables to remove the crosslinked and cooled cable from the pressurized cooling zone in a temperature higher than in the prior art, when measured from the conductor. Also preferably, the cooling may be effect at lower pressures compared to prior art.

Optionally, if desired, the crosslinked cable of the invention may be subjected to an additional non-pressurised cooling step after said pressurized cooling step, for further cooling of the cable.

The cable preparation process of the invention optionally comprises a further recovering step of the cable coming from the cooling step. Recovering may be effected by winding the cable on a cable drum in a known manner.

In a further embodiment of the process of the invention the cable obtained from the cooling step and optionally recovered, e.g. wound to a cable drum, may optionally be subjected, if needed in some applications, to a subsequent degassing step i.a. for removing or reducing any volatile decomposition products possibly resulting from said crosslinking step of the invention. In said degassing step the cable of the invention is preferably exposed either in ambient or elevated temperature for a period of time. As an example only, said degassing temperature may be e.g. 50-80°C, for a time period of one to four weeks. In one embodiment of the crosslinking process said degassing step may be shortened considerably or even avoided due to decreased level of said volatile by-products.

The cable of the invention produced by the above process of the invention may finally be further processed, e.g. protected with a protective layer, and/or optionally covered by a jacketing layer in a subsequent finishing step in a known manner and recovered for the end use thereof.

The invention thus provides also a crosslinked cable comprising a crosslinked polymer composition as defined above, preferably a crosslinked low voltage cable or power cable, more preferably a crosslinked power cable, as defined above. Preferably said crosslinked cable is obtainable by any of the crosslinking process as defined above.

In one embodiment of a crosslinking process of the invention a crosslinked power cable is produced which is selected from a crosslinked MV cable, wherein the lowest degree of crosslinking in a cable layer(s) meets the requirements as specified in IEC 60502, or a crosslinked HV cable, wherein the lowest degree of crosslinking in a cable layer(s) meets the requirements as specified in IEC 60840, which specifications are well known in the Wire & Cable field.

The advantageous compositions of the invention contain free radical generating agents which can be used for improving the quality of the products, e.g. in cable production processes. Due to the present invention the amount of voids in polymer products, such as cable layers can be reduced or even avoided, since less or no volatile decomposition products are formed. Moreover, the invention also enables improvement in the processability of a cable, i.a. in terms of safer and faster processing. E.g. the crosslinking process of the invention can be faster and/or more economical, since both cooling and/or degassing steps may be carried out in a reduced time and/or in a less energy consuming manner, if desired.

**Determination methods**

Unless otherwise stated the below determination methods were used to determine the properties defined generally in the description part and claims and in the experimental part.

**Melt Flow Rate**

The melt flow rate (MFR) is determined according to ISO 1133 and is indicated in g/10 min. The MFR is an indication of the flowability, and hence the processability, of the polymer. The higher the melt flow rate, the lower the viscosity of the polymer. The MFR is determined at 190 °C for polyethylenes and may be determined at different loadings such as 2.16 kg ($MFR_2$) or 21.6 kg ($MFR_{21}$). The MFR is determined at 230 °C for polypropylenes.

**Density**

The density was measured according to ISO 1183D. The sample preparation was executed according to ISO 1872-2.

**Amount of double bonds in the Polymer Composition or in the unsaturated polymer**

This method applies both for the Polymer Composition and for the unsaturated polymer (A). Both are referred below as a Composition or a polymer, respectively. The procedure for the determination of the amount of double bonds/ 1000 C-atoms is based upon the ASTM D3124-72 method. In that method, a detailed description for the determination of vinylidene groups/1000 C-atoms is given based on 2,3-dimethyl-1,3-butadiene. The described sample preparation procedure has also been applied for the determination of vinyl groups/1000 C-atoms, vinylidene groups/1000 C-atoms and trans-vinylene groups/1000 C-atoms in the present invention. However, for the determination of the extinction coefficient for these three types of double bonds, the following three compounds have been used: 1-decene for vinyl, 2-methyl-1-heptene for vinylidene and trans-4-decene for *trans*-vinylene, and the procedure as described in ASTM-D3124 section 9 was followed.
The total amount of double bonds was analysed by means of IR spectrometry and given as the amount of vinyl bonds, vinylidene bonds and *trans*-vinylene bonds per 1000 carbon atoms.

The composition or polymer to be analysed were pressed to thin films with a thickness of 0.5-1.0 mm. The actual thickness was measured. FT-IR analysis was performed on a Perkin Elmer 2000. Four scans were recorded with a resolution of 4 $cm^{-1}$.

A base line was drawn from 980 $cm^{-1}$ to around 840 $cm^{-1}$. The peak heights were determined at around 888 $cm^{-1}$ for vinylidene, around 910 $cm^{-1}$ for vinyl and around 965 $cm^{-1}$ for *trans*-vinylene. The amount of double bonds/ 1000 carbon atoms was calculated using the following formulas:

$$\text{vinylidene/1000 C-atoms} = (14 \times A)/(18.24 \times L \times D)$$

$$\text{vinyl/1000 C-atoms} = (14 \times A)/(13.13 \times L \times D)$$

$$\textit{trans}\text{-vinylene/1000 C-atoms} = (14 \times A)/(15.14 \times L \times D)$$

wherein
A: absorbance (peak height)
L: film thickness in mm
D: density of the material ($g/cm^3$)

The amount of vinyl groups originating from the polyunsaturated comonomer per 1000 carbon atoms was determined and calculated as follows:

The polymer to be analysed and a reference polymer have been produced on the same reactor, basically using the same conditions, i.e. similar peak temperatures, pressure and production rate, but with the only difference that the polyunsaturated comonomer is added to polymer to be analysed and not added to reference polymer. The total amount of vinyl groups of each polymer was determined by FT-IR measurements, as described above. Then, it is assumed that the base level of vinyl groups, i.e. the ones formed by the process and from chain transfer agents resulting in vinyl groups (if present), is the same for the reference polymer and the polymer to be analysed with the only exception that in the polymer to be analysed also a polyunsaturated comonomer is added to the reactor. This base level is then subtracted from the measured amount of vinyl groups in the polymer to be analysed, thereby resulting in the amount of vinyl groups/ 1000 C-atoms, which result from the polyunsaturated comonomer.

Calibration procedure for measuring the double bond content of an unsaturated low molecular weight compound (iii), if present (referred below as Compound)

The molar absorptivity for Compound (e.g. a crosslinking booster or a scorch retarder compound as exemplified in the descritpion part) can be determined according to ASTM D6248-98. At least three solutions of the Compound in $CS_2$ (carbon disulfide) are prepared. The used concentrations of the solutions are close to 0.18 mol/l. The solutions are analysed with FTIR and scanned with resolution 4 $cm^{-1}$ in a liquid cell with path length 0.1 mm. The maximum intensity of the absorbance peak that relates to the unsaturated moiety of the Compound(s) (each type of carbon-carbon double bonds present) is measured.

The molar absorptivity, B, in litres/molxmm for each solution and type of double bond is calculated using the following equation:
<u>B = (1/CL) x A</u>

C= concentration of each type of carbon-carbon double bond to be measured, mol/l
L= cell thickness, mm
A = maximum absorbance (peak height) of the peak of each type of carbon-carbon double bond to be measured, mol/l.

The average of the molar absorptivity, B, for each type of double bond is calculated. The average molar absorptivity, B, of each type of carbon-carbon double bond can then be used for the calculation of the concentration of double bonds in the reference polymer and the polymer samples to be analysed.

**Gel Content**

The gel content was determined according to ASTM D 2765-01, method A using a crosslinked sample which consists of the polymer composition under test and prepared according to "**Preparation of samples, crosslinking" below**.

Gel content on a cable is carried out usine ASTM D 2765-01 method B.

Methods A and B give comparable results.

**GC-Analysis protocol**

In definitions of the Compounds, Polymer compositions, cables and preparation process and modification methods thereof as defined above and in claims below, the volatile, e.g. $CH_4$, content given in ppm (weight) or as "absent" is determined by gas chromatography (GC) from a sample which is modified, e.g. crosslinked.

The test is used to determine the produced volatiles, e.g. methane, content of a free radical generating agent. The test free radical generating agent is used in such an amount with which a crosslinking degree expressed as gel content of 50 % was achieved, preferably gel content of at least 50%. Crosslinking conditions of the sample are not critical and may be effected e.g. as described below under

**"Preparation of samples, crosslinking"**

Volatiles are measured by taking a sample specimen of 1 g with a thickness of 1.5 mm from a modified, e.g. crosslinked composition, e.g. a plaque or cable. In the case of a cable comprising a crosslinked layer(s), the sample specimen is taken from a layer material of a crosslinked and cooled cable sample that is taken at the exit of a crosslinking/cooling zone, such as at the exit of a vulcanisation tube, after pressurised cooling step in a manner known for a skilled person.

The collection of volatiles from said sample specimen (to a head space bottle, see below) is started within one hour after the modification step is stopped, or in case of a crosslinked and cooled cable, within one hour after the cable sample is taken at the exit of a crosslinking/cooling zone.

A sample specimen of a thickness of 1.5 mm and of a weight of 1 g is cut from the middle of a plaque which is 100 mm wide and 100 mm long. The obtained sample (specimen) is placed in a 120 ml head space bottle with an aluminium crimp cup with seal and heat treated at 60 ° C for 1.5 h for collecting any gaseous volatiles present in said sample. Then 0.3-0.5 ml of the gas captured in the sample bottle is injected into a gas chromatograph, wherein the presence and content of the volatiles, e.g. methane, which are desired to be measured in a known manner. Double samples are analysed and a "zero-sample" without free radical generating agent/modification is used as a reference. The instrument used herein was a Varian 3400 with a $Al_2O_3/Na_2SO_4$ -column of 0,53mm x 50m, supplied by Chrompack.

**Specimen from a cable**

A sample specimen of a thickness of 1.5 mm and of a weight of 1 g is cut in an axial direction from said cable sample from the middle distance (in radial direction) of the polymer layer(s) ring surrounding the conductor of said cable sample (i.e. at the distance of ½ radius of said cable layer ring). The collection and determination of volatiles was carried out as described above.

**Specimen from a plaque**

The volatile, e.g. $CH_4$, content given in ppm (weight) or as "absent" is determined by gas chromatography (GC) from a sample plaque which is modified, e.g. crosslinked according to the protocol described in the section entitled "Preparation of samples, crosslinking" above. The test composition contains 2 parts test peroxide and 100 parts test polymer (i.e. sufficient to cause a degree of cross-linking of 50% or more).

A sample specimen of a thickness of 1.5 mm and of a weight of 1 g is cut from the middle of a plaque which

is 100 mm wide and 100 mm long. The collection and determination of volatiles was carried out as described above.

### Materials

In each test for references and for examples of this application the test arrangement for the reference polymer, i.e. the polymer without any added additive such as organic peroxide, and for the tested compositions, i.e. the reference polymer containing the organic peroxide, was the same.

The unsaturated polymer: The polymer is a poly (ethylene -co-1,7-octadiene)

### Poly (ethylene -co-1,7-octadiene) manufacture

Ethylene was compressed in a 5-stage precompressor and a 2-stage hyper compressor with intermediate cooling to reach an initial reaction pressure of ca. 2950 bar. The total compressor throughput was ca. 30 tons/hour. In the compressor area approximately 120 kg propylene / hour was added as chain transfer agent to maintain an MFR of 3.2g / 10 min. Here also 1,7-octadiene was added to the reactor in amount of ca. 50 kg/h. The compressed mixture was heated to approximately 165 °C in a preheating section of a front feed three-zone tubular reactor with an inner diameter of ca. 40 mm and a total length of ca. 1200 meters. A mixture of commercially available peroxide radical initiators dissolved in isododecane was injected just after the preheater in an amount sufficient for the exothermal polymerization reaction to reach peak temperature of ca. 280 °C after which it was cooled to approx 250 °C. The subsequent 2nd and 3rd peak reaction temperatures were ca. 280 °C and ca. 270 °C, respectively, with a cooling in between down to approximately 250 °C. The reaction mixture was depressurized by a kick valve, cooled and polymer was separated from unreacted gas.

The obtained polymer had a total number of C-C carbon double bonds of 1.286/1000 C and the number of vinyl groups was 0.994 vinyl groups/1000 C. The density of the material was 920 kg/$m^3$ and MFR(2.16 kg) = 3.2 g/10 min.

The above unsaturated polymer was used in testing the examples of the invention containing compounds (I) of the invention as the crosslinking agent, comparative examples with dicumulperoxide as the crosslinking agent and the reference example containing no crosslinking agent.

The commercial reference organic peroxide, dicumyl peroxide, was supplied by AkzoNobel.

### Preparation of samples, impregnation

The test polyethylene pellets were ground to a fine powder in a Retsch grinder with a 1.5 mm sieve. The powder obtained was impregnated with the test peroxide dissolved in a pentane solution until the pentane had evaporated to give a dry powder of the test peroxide and the test polymer. The content of the test composition was 3 parts test peroxide and 100 parts test polymer when the gel content of the crosslinked test composition was tested as described below. The content of the test composition was 2 parts test peroxide and 100 parts test polymer when the volatiles content was determined as described in the GC-analysis protocol.

### Preparation of samples, crosslinking

The test plaques had the following dimensions and crosslinking cycle. The plaques were 100 mm long, 100 mm wide, and 0.1 mm thick when used for determination of the gel content as described below, and 100 mm long, 100 mm wide, and 1.5 mm thick when the volatiles content was determined as described in the GC-analysis protocol below. The crosslinking was conducted in a Specac press, where the composition was kept at 120 °C for 1 min at 5 bar, then the temperature was increased with 60 °C/min for 1 min to reach 180 °C at 5 bar, and kept at 180 °C at 5 bar for 12 min, followed by cooling to ambient temperature over 30 min at 5 bar.

## EXAMPLES

### Example 1

### Preparation of Di-(1-methyl-1-phenylundecyl) peroxide

**($R^1$, $R^1$, = phenyl; $R^2$, $R^2$, = methyl; $R^3$, $R^3$, = decyl)**

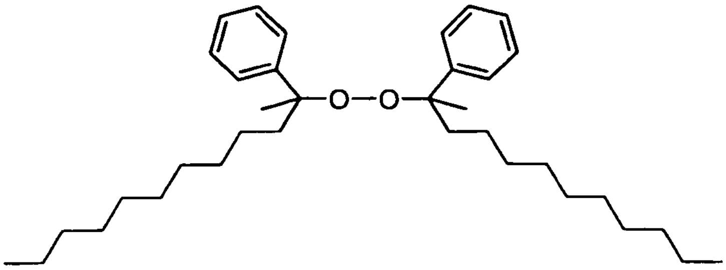

(Ib)

A. 1-methyl-1-phenylundecyl alcohol

To a suspension of 2.43 g (0.1 mol) magnesium turnings in 10 ml of diethyl ether was added 0,1 ml of 1,2-dibromoethane and the mixture was stirred. 22.17 g (0.1 mol) of 1-bromodecane in 20 ml diethyl ether was added dropwise and the mixture was refluxed for 15 minutes, then cooled. 9.61 g (0.08 mol) of acetophenone in 20 ml diethyl ether was added while cooling on ice bath. The ice bath was removed and the reaction mixture stirred at room temperature for 30 minutes. The mixture was then poured into a slurry of 30 g ammonium chloride in 150 ml water and 100 g ice while stirring vigorously. The mixture was filtered, the ether layer separated and the aqueous layer extracted twice with 50 ml of ether. The organic layers were combined, washed with water, 10% $NaHSO_3$, brine, dried and evaporated to give 22.48 g of clear oil. The oil was purified with dry column chromatography using pentane. The eluant was evaporated giving 17.22 g (82%) of 1-methyl-1-phenylundecyl alcohol as a viscous colorless oil.

B. 1-methyl-1-phenylundecyl hydroperoxide

10.50 g (0.04 mol) of 1-methyl-1-phenylundecyl alcohol was dissolved in 50 ml of dichloromethane, cooled in ice bath, 10.6 ml (12.29 g, 0.08 mol) of trimethylsilyl bromide was added and the mixture stirred for 1 h under protection from moisture. The solution was diluted with 100 ml ether and washed four times with 50 ml water, brine, dried and evaporated to give crude 2-phenyl-2-bromododecane. 35 ml of 2.3 M hydrogen peroxide in THF (0.08 mol) was added to the 2-phenyl-2-dodecyl bromide and the mixture was cooled on ice bath. 8.84 g (0.04 mol) of silver trifluoroacetate was added. 70 ml of conc. $NaHCO_3$ was added and the mixture filtered. The reaction flask and the filter cake was rinsed with diethyl ether. The aqueous phase was separated and the organic phase washed with conc. $NaHCO_3$, 50 ml water, brine, dried and evaporated to give an oil. The oil was purified by flash chromatography using 2:8 ether:pentane as eluent. The yield of 1-methyl-1-phenylundecyl hydroperoxide was 30%.

C. Di-(1-methyl-1-phenylundecyl) peroxide

0.942 g (3.6 mmol) of 1-methyl-1-phenylundecyl alcohol was dissolved in 5 ml of dichloromethane, 1 ml of trimethylsilyl bromide (7.2 mmol) was added and the mixture stirred for 1 h under protection from moisture. The solution was diluted with 15 ml of diethyl ether, washed with water (3x10 ml), 15 ml brine, dried and evaporated to give 1.18 of crude 2-phenyl-2-bromododecane. 795 mg of silver trifluoroacetate (3.6 mmol) was dissolved in 5 ml THF. To the crude bromide was added 500 mg of 1-methyl-1-phenylundecyl hydroperoxide (1.8 mmol) dissolved in 10 ml THF. This mixture was cooled in ice-salt bath to -15°C and the silver trifluoroacetate solution added with a pipette. 2 ml of brine was then added, followed by 10 ml conc. $NaHCO_3$. The reaction mixture was stirred and filtered. The reaction flask and the filter cake were rinsed with 15 ml diethyl ether. The aqueous phase was separated and the organic phase washed with conc. $NaHCO_3$, 15 ml water, 15 ml brine, dried and evaporated to give 1.40 g of a yellowish oil. Purification was done using a 1:9 ether:pentane mixture as eluent. The yield was 409 mg (43%). $^{13}C$-NMR ($CDCl_3$) δ 14.33, 22.91, 23.96, 24.06, 24.19, 29.55, 29.72, 29.82, 30.24, 32.13, 40.68, 40.97, 84.18, 126.16, 126.69, 127.86, 145.59, 145.71

**Example 2**

**Preparation of Di(1-methyl-cyclohexyl) peroxide**

**($R^1$ = methyl; $R^2$ + $R^3$ form together with $C^1$ a cyclohexyl ring and $R^{1'}$ = methyl; $R^{2'}$ + $R^{3'}$ form together with $C^{1'}$ a cyclohexyl ring)**

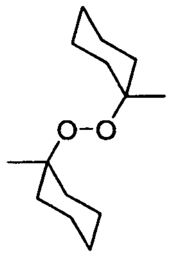

A. Di(1-methyl-cyclohexyl)peroxide

1-methylcyclohexanol (30 g, 0.26 mol) was placed in a 100mL three necked round bottomed flask and was stirred. The flask was cooled in a brine/ice bath, dropping funnel fitted and fitted with a static $N_2$ supply. The dropping funnel was charged with 98% sulfuric acid (16.14 ml) and water (6.45 ml) giving a 70% sulfuric acid solution. This was added dropwise to the 1-methylcyclohexanol and stirring continued to give a viscous brown mixture. The bath was recharged with ice/brine, dropping funnel rinsed with water and recharged with 35% hydrogen peroxide (6.98mL, 0.125mol) and added dropwise. The solution separated into two phases. Cyclohexanol (150mL) was added and the mixture was transferred to a separating funnel. The aqueous fraction was extracted with another portion of cyclohexane (150ml) and the combined organic fractions washed with 1 M NaOH (2x100mL), water (2x150mL), dried and evaporated to give a viscous colourless oil. (12.98g). The oil was sorbed onto silica gel then placed on a silica gel column and eluted with cyclohexane. After evaporation at reduced pressure 0.7 g colourless oil of di(1-methyl-cyclohexyl) peroxide was obtained. $^{13}$C-NMR ($CDCl_3$) $\delta$ 22.45, 25.01, 25.95, 35.39, 78.58

**Example 3**

**Preparation of Di(1-methyl-cyclopentyl) peroxide**

($R^1$ = methyl; $R^2$ + $R^3$ form together with $C^1$ a cyclopentyl ring and $R^{1'}$ = methyl; $R^{2'}$ + $R^{3'}$ form together with $C^{1'}$ a cyclopentyl ring)

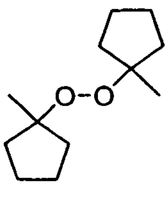

A. Di(1-methyl-cyclopentyl) peroxide

A 250 ml tri-neck round bottom flask was equipped and a 50 ml addition funnel and the flask was cooled <0C. 30g of 1-methylcyclopentanol (0.3 M, 1 EQ) was added to the flask. 70% $H_2SO_4$ solution was prepared and cooled in an ice bath. The $H_2SO_4$ (12.71 ml, 0.91 M, 3 EQ) was added drop wise over 15 minutes and the reaction mixture was stirred for ~2.5 hours in order to allow all the 1-methylcyclopentanol to dissolve. With the reaction stirring, 8.11ml of $H_2O_2$ 35% (wt) (0.24 M, 0.8 EQ) was added drop wise over 15 minutes. The reaction was left stirring overnight. The

reaction mixture was transferred to a separation funnel and extracted three times with 50 ml of pentane each time. Organic layers were collected and the aqueous was set aside. The organic layers were extracted 3 times with 50 ml of 1N NaOH each time to remove excess acid. The organic layer was collected, dried and concentrated. The residue was purified by chromatography on a silica column using pentane as the mobile phase. The product fractions were concentrated to yield 973 mg of di(1-methyl-cyclopentyl) peroxide as a colorless oil. $^{13}C$-NMR ($CDCl_3$) δ 24.43, 24.75, 37.13, 89.23

**Gel content**

The gel content of the LDPE copolymer prepared as described above was determined according to the method above and the results are shown below (Table 1.)

**Table 1.** Gel content

| Example | Gel content (%) |
|---|---|
| Reference polymer without peroxide | 0 |
| Ib | 51 |
| Ia | 62 |
| Ia' | 82 |

**GC-Analysis**

GC-analysis was performed to evaluate the amount of formed $CH_4$. The example is compared to a sample using dicumyl peroxide, which represent the conventional solution used today. The results are presented below (Table 2).

**Table 2**. GC-analysis of the $CH_4$ content.

| Example | $CH_4$ content (ppm) |
|---|---|
| Dicumyl peroxide | 719 (gel content 93%) |
| Ia' | <5* (gel content 84%)* |
| * at values less than 5 ppm the amount of methane is so small that noise masks an accurate reading. Value less than 5ppm are considered to represent non methane formed therefore. | |

**Preparation example of the crosslinked cable of the invention:**

A power cable comprising an inner semiconductive layer, an insulation layer and an outer semiconductive layer for experimental testing is prepared by coextruding on a conductor said layers in given order using a conventional extruder line and conventional extrusion conditions.

The layer materials are conventional polymer grades and each layer comprises a peroxide compound of the invention as a crosslinking agent.

The semiconductive material used in the cable, both as inner and outer semicon, is a poly(ethylene-co-butylacrylate) polymer (with a butylacrylate content of 17wt%) containing 40 wt% of a furnace black. The composition is stabilised with an antioxidant of the polyquinoline type and contains 1 wt% of the peroxide of the invention as a crosslinking agent.

The middle insulation layer is formed of low density polyethylene LDPE ($MFR_2$=2 g/10 min) containing 2 wt-% of the peroxide of the invention and 0.2 wt-% of 4,4'-thiobis(2-tert.-butyl-5-methylphenol).

The obtained cable is immediately after extrusion subjected to a conventional vulcanisation tube and crosslinked in a known manner using well known crosslinking conditions. After crosslinking the cable is then cooled in cooling zone of said vulcanisation tube at a desired pressure and temperature. The cooling step is stopped when the desired temperature measured from the conductor is achieved. Typically the cooling step can be effected in a lower pressure and/or the cooling step can be stopped in a higher temperature at conductor compared to corresponding cable crosslinked to a same gel content, but using dicumylperoxide as the crosslinking agent. The crosslinked and cooled layer is wound to

a cable drum and transferred to a degassing step to remove the volatile(s) content, if any. This step can typically be done in a lower temperature and/or a shorter period compared to corresponding cable crosslinked to a same gel content, but using dicumylperoxide as the crosslinking agent.

## Claims

**1.** A polymer composition comprising

A) an unsaturated polymer, and
B) a free radical generating compound

- wherein the free radical generating compound is a compound of formula (I)

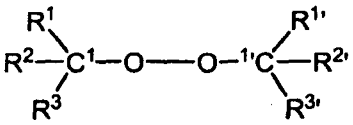
(I)

wherein
- $R^1$ and $R^{1'}$ are each independently H, substituted or unsubstituted saturated or partially unsaturated hydrocarbyl; or substituted or unsubstituted aromatic hydrocarbyl;

- wherein each of said substituted or unsubstituted saturated or partially unsaturated hydrocarbyl or aromatic hydrocarbyl optionally comprises one or more heteroatoms;

and

- wherein said substituted saturated or partially unsaturated hydrocarbyl or substituted aromatic hydrocarbyl comprises independently 1 to 4 substituents selected from a functional group, or saturated or partially unsaturated hydrocarbyl optionally bearing a functional group; or aromatic hydrocarbyl optionally bearing a functional group;

- $R^2$, $R^{2'}$, $R^3$ and $R^{3'}$ are each independently H, substituted or unsubstituted saturated or partially unsaturated hydrocarbyl; or substituted or unsubstituted aromatic hydrocarbyl;

- wherein each of said substituted or unsubstituted saturated or partially unsaturated hydrocarbyl or aromatic hydrocarbyl optionally comprises one or more heteroatoms; and
- wherein said substituted saturated or partially unsaturated hydrocarbyl or substituted aromatic hydrocarbyl comprises independently 1 to 4 substituents selected from a functional group, a saturated or partially unsaturated hydrocarbyl optionally bearing a functional group; or an aromatic hydrocarbyl optionally bearing a functional group; or

-$R^2$ and $R^3$ together with the carbon atom ($C^1$) to which they are attached form an unsubstituted or substituted, saturated or partially unsaturated carbocyclic ring moiety of 3 to 14 C-atoms; unsubstituted or substituted saturated or partially unsaturated heteroring moiety of 3 to 14 ring atoms comprising 1 to 6 heteroatoms selected from O, N, P, S or Si; or unsubstituted or substituted aromatic ring moiety of 3 to 14 C-atoms optionally comprising 1 to 4 heteroatoms;

- wherein said carbocyclic ring, heteroring or aromatic ring system is optionally fused with another ring system having 4 to 14 ring atoms; and
- wherein said substituted carbocyclic ring, heteroring or aromatic ring system comprises 1 to 4 substituents selected independently from a functional group, saturated or partially unsaturated hydrocarbyl optionally bearing a functional group; or an aromatic hydrocarbyl optionally bearing a functional group; or

-$R^{2'}$ and $R^{3'}$ to gether with the carbon atom ($C^{1'}$) to which they are attached form an -$R^2_{,}$ and $R^{3'}$ together with

the carbon atom ($C^{1'}$) to which they are attached form an unsubstituted or substituted saturated or partially unsaturated carbocyclic ring moiety of 3 to 14 C-atoms; unsubstituted or substituted saturated or partially unsaturated heteroring moiety of 3 to 14 ring atoms comprising 1 to 6 heteroatoms selected from O, N, P, S or Si; or unsubstituted or substituted aromatic ring moiety of 3 to 14 C-atoms optionally comprising 1 to 4 heteroatoms;

- wherein said carbocyclic ring, heteroring or aromatic ring system is optionally fused with another ring system having 4 to 14 ring atoms; and
- wherein said substituted carbocyclic ring, heteroring or aromatic ring system comprises 1 to 4 substituents selected independently from a functional group, a saturated or partially unsaturated hydrocarbyl optionally bearing a functional group; or an aromatic hydrocarbyl optionally bearing a functional group; or

- $R^2$ and $R^{2'}$ form together a bivalent substituted or substituted saturated or partly unsaturated hydrocarbyl optionally containing 1 to 4 heteroatoms, wherein $R^2$ is linked to $C^1$ and $R^{2'}$ to $C^{1'}$, respectively, forming together with -$C^1$-O-O-$C^{1'}$- a substituted or unsubstituted saturated or partially unsaturated carbocyclic ring moiety of 3 to 14 C-atoms comprising optionally, in addition to said at least two O atoms, 1 to 4 further heteroatoms; wherein said carbocyclic ring or heteroring system is optionally fused with another ring system having 4-14 ring atoms;
or functional derivatives thereof;
- with a proviso that at least two of $R^1$, $R^2$ and $R^3$, and at least two of $R^{1'}$, $R^{2'}$ and $R^{3'}$, respectively, are other than H or methyl.

**2.** A polymer composition as defined in claim 1 comprising

A) an unsaturated polymer, and
B) a free radical generating compound

- wherein the polymer composition and/or the unsaturated polymer contains carbon-carbon double bonds in an amount of at least 0-05 e.g. 0.1 or more, more preferably of 0.2 or more, and most preferably more than 0.37 carbon-carbon double bonds/1000 carbon atoms, and
- wherein the free radical forming agent is a compound of formula (I) as defined in claim 1.

**3.** A compound of formula (1) as defined in claim 1 to 2, wherein $R^2$ and $R^3$ together with carbon atom ($C^1$) to which they are attached from an optionally substituted carbocyclic ring moiety of 3 to 12 ring C-atoms or an optionally substituted heteroring moiety of 3 to 12 ring atoms containing 1 to 6, preferably 1 to 4, heteroatoms selected from O, N, P, S or Si, and wherein said carbocyclic or heterocyclic ring system is optionally fused with another ring system having 4 to 14 ring atoms, preferably $R^2$ and $R^3$ together with carbon atom ($C^1$) form a (C3-C12) carbocyclic ring moiety; and
wherein $R^{2'}$ and $R^{3'}$ together with carbon atom ($C^{1'}$) to which they are attached form an optionally substituted carbocyclic ring moiety of 3 to 12 ring C-atoms or an optionally substituted heteroring moiety of 3 to 12 ring atoms containing 1 to 6, preferably 1 to 4, heteroatoms selected from O, N, P, S or Si, and wherein said carbocyclic or heterocyclic ring system is optionally fused with another ring system having 4 to 14 ring atoms, preferably $R^{2'}$ and $R^{3'}$ together with carbon atom ($C^{1'}$) form a (C3-C12) carbocyclic ring moiety; preferably
wherein $R^2$ and $R^3$ together with carbon atom ($C^1$) to which they are attached and/or wherein $R^{2'}$ and $R^{3'}$ together with carbon atom ($C^{1'}$) to which they are attached form an optionally substituted, saturated or partially unsaturated mono- or bicyclic (C4-C14) carbocyclic ring, more preferably an optionally substituted, saturated monocyclic (C5-C8)carbocyclic ring, especially
wherein $R^2$ and $R^{2'}$ are the same radical and, $R^3$ and $R^{3'}$ are the same radical.

**4.** A compound of formula (I) as defined in any of the preceding claims, wherein the ring system formed by $R^{2'}$ and $R^{3'}$ together with the carbon atom ($C^{1'}$) to which they are attached is same as the ring system formed by $R^2$ and $R^3$ together with the carbon atom ($C^1$) to which they are attached; and

- wherein $R^1$ and $R^{1'}$ each represents optionally substitution branched or straight chain, preferably unsubstituted straight chain, (C2-C30)alkyl (preferably (C6-C30)alkyl) or methyl, preferably wherein $R^1$ and $R^{1'}$ are identical; preferably
- wherein $R^2$ and $R^3$ together with carbon atom ($C^1$) to which they are attached form an optionally substituted carbocyclic ring moiety of 3 to 12 ring C-atoms which is optionally fused with another ring system having 4 to

14 ring atoms, and
- wherein $R^{2'}$ and $R^{3'}$ together with carbon atom ($C^{1'}$) to which they are attached form an optionally substituted carbocyclic ring moiety of 3 to 12 ring C-atoms which is optionally fused with another ring system having 4 to 14 ring atoms, and
- wherein the ring system formed by $R^{2'}$ and $R^{3'}$ together with the carbon atom ($C^{1'}$) to which they are attached is the same as the ring system formed by $R^2$ and $R^3$ together with the carbon atom ($C^1$) to which they are attached and,
- wherein $R^1$ and $R^{1'}$ each represents optionally substituted branched or straight chain, preferably unsubstituted straight chain, (C6-C30)alkyl or methyl.

**5.** A compound of formula (I) as defined in claim 1 to 4, wherein $R^1$, $R^2$, $R^3$, $R^1$, $R^{2'}$ and $R^{3'}$ each independently is optionally substituted mono- or multicyclic (C5-C14)aryl; optionally substituted mono- or multicyclic (C5-C14) heteroaryl; optionally substituted mono- or multicyclic ($C_{4-}C_{14}$)cycloalkyl; optionally substituted mono- or multicyclic (C4-C14)heterocyclyl; optionally substituted straight or branched chain (C1-C50)alkyl, preferably optionally substituted straight chain (C1-C30)alkyl; optionally substituted straight or branched chain (C1-C50)alkenyl or optionally substituted straight or branched chain (C1-C50)alkynyl, preferably straight chain (C1-C30)alkenyl or straight chain (C1-C30)alkynyl; optionally substituted straight or branched chain (C1-C50)heteroalkyl comprising 1 to 4 heteroatoms selected from O, N, P, S or Si,; preferably
wherein $R^2$, $R^{2'}$, $R^3$ and $R^{3'}$ are independently selected from unsubstituted straight chain (C1-C50)alkyl, preferably (C1-C30)alkyl, more preferably (C1-C20)alkyl, more preferably from C1-C12alkyl, more preferably from methyl or (C6-C12)alkyl.

**6.** A compound of formula (I) as defined in claim 5, herein

- $R^2$ and $R^{2'}$ are same and each represents methyl; or
- $R^2$ and $R^{2'}$ are same and each represents (C6-C30)alkyl and/or

wherein $R^3$ and $R^{3'}$ are same and each represents (C6-C30)alkyl.

**7.** A compound of formula (I) as defined in any of the preceding claims, selected from wherein

- $R^1$ and $R^{1'}$ are same or different, preferably same, and each represents optionally substituted, saturated or partially unsaturated cyclic hydrocarbyl of 5 to 14 ring atoms optionally containing 1 to 4 heteroring atoms selected from N, O, P, S or Si; or optionally substituted mono- or multicyclic (C5-C14)aryl, preferably unsubstituted monocyclic (C5-C7)aryl; or
- $R^1$ and $R^{1'}$ are same or different preferably same and each represents optionally substituted branched or straight chain, preferably unsubstituted straight chain, (C6-C30)alkyl or methyl.

**8.** A compound of formula (I) as claimed in any of the preceding claims wherein $R^1$ and $R^{1'}$ are same and each represents methyl; and
$R^2$ and $R^3$ together with $C^1$ atom to which they are attached form an optionally substituted, saturated or partially unsaturated mono- or bicyclic (C4-C14)carbocyclic ring, preferably unsubstituted saturated monocyclic (C5-C8) carbocyclic ring;
and $R^{2'}$ and $R^{3'}$ together with the carbon atom ($C^{1'}$) to which they are attached form an optionally substituted, saturated or partially unsaturated mono- or bicyclic (C4-C14)carbocyclic ring, preferably unsubstituted saturated monocyclic (C5-C8)carbocyclic ring; whereby the ring system formed by $R^2$ and $R^3$ together with $C^1$ is preferably identical to a ring system formed by $R^{2'}$ and $R^{3'}$ together with $C^{1'}$.

**9.** A compound as defined in claim 8 of formula (II)

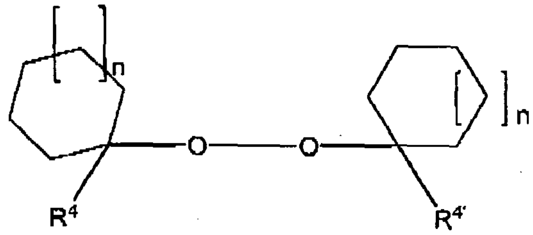

(II)

wherein n is 0 to 3, and $R^4$ and $R^{4'}$ each independently represent a straight chain alkyl group having 1 to 30 carbon atoms, preferably methyl or straight chain alkyl group having 6 to 20, preferably 6 to 12, carbon atoms, more preferably methyl, and wherein one or both ring systems independently are unsubstituted or optionally substituted by 1 to 4 substituents.

**10.** A compound as defined in any of the preceding claims 4 to 9, wherein $R^1$ and $R^{1'}$ are both same and represent an optionally substituted, preferably unsubstituted, monocyclic (C5-C7)aryl;
$R^2$ and $R^{2'}$ are same and are both methyl; and
$R^3$ and $R^{3'}$ are same and are both optionally substituted branched or straight chain (C6-C50)alkyl, more preferably unsubstituted straight chain (C6-C30)alkyl, such as (C6-C20)alkyl.

**11.** A compound as claimed in claim 10 of formula (III)

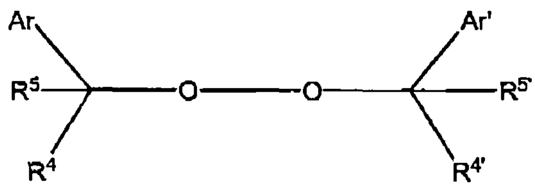

wherein Ar and Ar' independently represent a phenyl, benzyl or naphthyl group optionally substituted by 1 to 4 substituents,
$R^4$ and $R^{4'}$ each are methyl; and
$R^5$ and $R^{5'}$ each independently represent a straight chain alkyl group having C6-30 carbon atoms, preferably 6 to 20, more preferably 6 to 12, carbon atoms.

**12.** A compound of formula (I) as defined in any of the preceding claims, wherein said optional substitutents are each independently selected from -OH, $-NR_2$, wherein each R is independently H or (C1-C12)alkyl, COR", wherein R" is i.a. H, (C1-C12)alkyl or $-NR_2$, wherein each R is as defined for $-NR_2$, COOR", wherein R is as defined for -COR"; halogen, such as -F, -Cl or -I; or alkoxy, saturated or partially unsaturated hydrocarbyl optionally bearing a functional group; or aromatic hydrocarbyl optionally bearing a functional group.

**13.** A compound as claimed in claim 1 of formula (V)

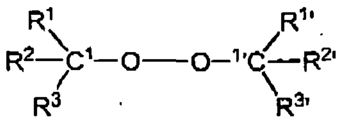

(V)

wherein the compounds are selected from any of the alternatives (i) to (iii):

(i) - $R^1$ and $R^{1'}$ are each independently H, substituted or unsubstituted saturated or partially unsaturated hydrocarbyl;

- wherein each of said substituted or unsubstituted saturated or partially unsaturated hydrocarbyl optionally comprises one or more heteroatoms;
- wherein said substituted or unsubstituted saturated or partially unsaturated hydrocarbyl include (i) straight or branched chain saturated or partially unsaturated hydrocarbyls, (ii) straight or branched chain saturated or partially unsaturated hydrocarbyls which bear saturated or partially unsaturated cyclic hydrocarbyl and (iii) saturated or partially unsaturated cyclic hydrocarbyls;
- wherein each of said saturated or partially unsaturated cyclic hydrocarbyl is independently a monocyclic or multicyclic ring system; and
- wherein said substituted saturated or partially unsaturated hydrocarbyl comprise independently 1 to 4 substituents selected from a functional group, a saturated or partially unsaturated hydrocarbyl optionally bearing a functional group or aromatic hydrocarbyl optionally bearing a functional group; and
- $R^2$, $R^{2'}$, $R^3$ and $R^{3'}$ are each independently as defined above for $R^1$ and $R^{1'}$; or

(ii) - $R^1$ and $R^{1'}$ are each independently an optionally substituted, preferably unsubstituted, monocyclic (C5-C7) aryl, preferably phenyl,

- wherein said substituted monocyclic (C5-C7)aryl comprises independently 1 to 4 substituents selected from a functional group, a saturated or partially unsaturated hydrocarbyl optionally bearing a functional group or aromatic hydrocarbyl optionally bearing a functional group; and

$R^2$ and $R^{2'}$ are same and are both methyl; and
$R^3$ and $R^{3'}$ are each independently H, substituted or unsubstituted saturated or partially unsaturated hydrocarbyl as defined above under (i) for $R^1$ and $R^{1'}$; or
(iii)
$R^1$ and $R^{1'}$ are each independently H, substituted or unsubstituted saturated or partially unsaturated hydrocarbyl as defined above under (i) for $R^1$ and $R^{1'}$; and

- $R^2$ and $R^3$ together with the carbon atom ($C^1$) to which they are attached form an unsubstituted or substituted saturated or partially unsaturated carbocyclic ring moiety of 3 to 14 C-atoms, preferably of 5 to 12 C atoms; or an unsubstituted or substituted saturated or partially unsaturated heteroring moiety of 3 to 14 ring atoms comprising 1 to 6, preferably 1 to 4 heteroatoms, selected from O, N, P, S or Si;

  - wherein said carbocyclic ring or heteroring is optionally fused with another optionally substituted ring system having 4 to 14 ring atoms; and
  - wherein said substituted carbocyclic ring or heteroring system comprises 1 to 4 substituents selected independently from a functional group, or a saturated or partially unsaturated hydrocarbyl optionally bearing a functional group; and

- $R^2$, and $R^{3'}$ together with the carbon atom ($C^{1'}$) to which they are attached form an unsubstituted or substituted saturated or partially unsaturated carbocyclic ring moiety of 3 to 14 C-atoms, preferably of 5 to 12 C atoms; an unsubstituted or substituted saturated or partially unsaturated heteroring moiety of 3 to 14 ring atoms comprising 1 to 6, preferably 1 to 4 heteroatoms, selected from O, N, P, S or Si;

  - wherein said carbocyclic ring or heteroring system is optionally fused with another optionally substituted ring system having 4 to 14 ring atoms; and
  - wherein said substituted carbocyclic ring or heteroring system comprises 1 to 4 substituents selected independently from a functional group or a saturated or partially unsaturated hydrocarbyl optionally bearing a functional group,
  - with a proviso for alternatives (i) to (iii) that at least two of $R^1$, $R^2$ and $R^3$, and at least two of $R^{1'}$, $R^{2'}$ and $R^{3'}$, respectively, are other than H or methyl.

**14.** A compound as defined in claim 1 to 2 which is selected from any of

- Di(1-methylcyclopentyl) peroxide
- Di-(1-methyl-1-phenylundecyl) peroxide
- Di-(1-methyl-1-phenylheptyl) peroxide or
- Di(1-methyl-cyclohexyl) peroxide.

**15.** A compound as claimed in any preceding claim which bears one or more moieties in its structure which are decomposable to a decomposition product in a free radical generating step

- wherein said one or more decomposable moieties result in a $CH_4$ content of less than 300 ppm (weight), preferably of less than 200 ppm (weight), preferably of less than 100 ppm (weight), more preferably of 0 to 50 ppm (weight), when determined according to a method as described in the description under "GC-analysis protocol"; or
- a compound without any such moiety that is decomposable to $CH_4$ as said decomposition product; or any mixture thereof.

**16.** A composition as claimed in any preceding claim wherein the unsaturated polymer is an unsaturated LDPE homopolymer or LDPE copolymer with one or more polyunsaturated comonomer(s), preferably one or more diene(s), especially one in the form of (1) polymer powder, (2) polymer pellets or (3) a mixture of a polymer melt, whereby said (1) polymer powder or, preferably, (2) polymer pellets are optionally contained in a container.

**17.** A modified polymer composition in which the polymer composition of any preceding claim is cross-linked by initiating a radical reaction in the polymer composition.

**18.** A crosslinkable cable which comprises a conductor which is surrounded by one or more layers, e.g. an insulation layer, jacketing layer and/or semiconductive layer comprising a polymer composition as claimed in 1 to 16 or cross-linked cable in which said polymer composition has been cross-linked by initiating a radical reaction in the polymer composition, preferably

- a low voltage cable comprising a conductor surrounded by an insulation layer and optionally a jacketing layer, wherein said insulation layer comprises a polymer composition as defined in claim 1 to 16; or
- a power cable comprising an electrical conductor surrounded by one or more layers comprising at least an inner semiconductive layer, insulation layer and an outer semiconductive layer, in that order, and optionally surrounded by a jacketing layer, wherein at least one of said layers comprises, a polymer composition as defined in claim 1 to 16.

## Patentansprüche

**1.** Polymerzusammensetzung, die folgendes aufweist:

A) ein ungesättigtes Polymer und
B) eine Radikale bildende Verbindung,

- wobei die Radikale bildende Verbindung eine Verbindung der Formel (I) ist

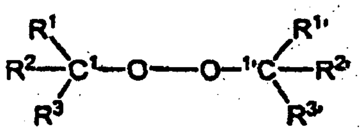

(I)

worin
- $R^1$ und $R^{1'}$ jeweils unabhängig voneinander H, ein substituierter oder unsubstituierter gesättigter oder teilweise ungesättigter Kohlenwasserstoffrest oder ein substituierter oder unsubstituierter aromatischer Kohlenwasserstoffrest sind;

- wobei jeder der substituierten oder unsubstituierten gesättigten oder teilweise ungesättigten Kohlenwasserstoffreste oder aromatischen Kohlenwasserstoffreste gegebenenfalls ein oder mehrere Heteroatome aufweist;
und
- wobei der substituierte gesättigte oder teilweise ungesättigte Kohlenwasserstoffrest oder substituierter aromatischer Kohlenwasserstoffrest unabhängig voneinander 1 bis 4 Substituenten aufweist, die ausgewählt sind aus einer funktionellen Gruppe oder einem gesättigten oder teilweise ungesättigten Kohlenwasserstoffrest, der gegebenenfalls eine funktionelle Gruppe aufweist, oder einem aromatischen Kohlenwasserstoffrest, der gegebenenfalls eine funktionelle Gruppe aufweist;

- $R^2$, $R^{2'}$, $R^3$ und $R^{3'}$ jeweils unabhängig voneinander H, ein substituierter oder unsubstituierter gesättigter oder teilweise ungesättigter Kohlenwasserstoffrest oder ein substituierter oder unsubstituierter aromatischer Kohlenwasserstoffrest sind;

  - wobei jeder der substituierten oder unsubstituierten gesättigten oder teilweise ungesättigten Kohlenwasserstoffreste oder aromatischen Kohlenwasserstoffreste gegebenenfalls ein oder mehrere Heteroatome aufweist; und
  - wobei der substituierte gesättigte oder teilweise ungesättigte Kohlenwasserstoffrest oder substituierte aromatische Kohlenwasserstoffrest unabhängig voneinander 1 bis 4 Substituenten aufweist, die ausgewählt sind aus einer funktionellen Gruppe, einem gesättigten oder teilweise ungesättigten Kohlenwasserstoffrest, der gegebenenfalls eine funktionelle Gruppe aufweist, oder einem aromatischen Kohlenwasserstoffrest, der gegebenenfalls eine funktionelle Gruppe aufweist; oder

- $R^2$ und $R^3$ zusammen mit dem Kohlenstoffatom ($C^1$), an das sie gebunden sind, eine unsubstituierte oder substituierte, gesättigte oder teilweise ungesättigte carbocyclische Ring-Einheit mit 3 bis 14 C-Atomen, eine unsubstituierte oder substituierte gesättigte oder teilweise ungesättigte Heteroring-Einheit mit 3 bis 14 Ringatomen, die 1 bis 6 Heteroatome aufweist, die aus O, N, P, S oder Si ausgewählt sind, oder eine unsubstituierte oder substituierte aromatische Ring-Einheit mit 3 bis 14 C-Atomen bilden, die gegebenenfalls 1 bis 4 Heteroatome aufweist;

  - wobei das carbocyclische Ring-, Heteroring- oder aromatische Ring-System gegebenenfalls mit einem weiteren Ring-system mit 4 bis 14 Ringatomen kondensiert ist; und
  - wobei das substituierte carbocyclische Ring-, Heteroring-oder aromatische Ring-System 1 bis 4 Substituenten aufweist, die unabhängig voneinander ausgewählt sind aus einer funktionellen Gruppe, einem gesättigten oder teilweise ungesättigten Kohlenwasserstoffrest, der gegebenenfalls eine funktionelle Gruppe aufweist, oder einem aromatischen Kohlenwasserstoffrest, der gegebenenfalls ein funktionelle Gruppe aufweist; oder

- $R^{2'}$ und $R^{3'}$ zusammen mit dem Kohlenstoffatom ($C^{1'}$), an das sie gebunden sind, eine unsubstituierte oder substituierte, gesättigte oder teilweise ungesättigte carbocyclische Ring-Einheit mit 3 bis 14 C-Atomen, eine unsubstituierte oder substituierte, gesättigte oder teilweise ungesättigte Heteroring-Einheit mit 3 bis 14 Ringatomen, die 1 bis 6 Heteroatome aufweist, die aus O, N, P, S oder Si ausgewählt sind, oder eine unsubstituierte oder substituierte aromatische Ring-Einheit mit 3 bis 14 C-Atomen bilden, die gegebenenfalls 1 bis 4 Heteroatome aufweist;

  - wobei das carbocyclische Ring-, Heteroring- oder aromatische Ring-System gegebenenfalls mit einem weiteren Ringsystem mit 4 bis 14 Ringatomen kondensiert ist; und
  - wobei das substituierte carbocyclische Ring-, Heteroring- oder aromatische Ring-System 1 bis 4 Substituenten aufweist, die unabhängig voneinander aus einer funktionellen Gruppe, einem gesättigten oder teilweise ungesättigten Kohlenwasserstoffrest, der gegebenenfalls eine funktionelle Gruppe aufweist, oder einem aromatischen Kohlenwasserstoffrest ausgewählt sind, der gegebenenfalls eine funktionelle Gruppe aufweist; oder

- $R^2$ und $R^{2'}$ zusammen einen zweiwertigen substituierten oder unsubstituierten gesättigten oder teilweise ungesättigten Kohlenwasserstoffrest bilden, der gegebenenfalls 1 bis 4 Heteroatome aufweist, wobei $R^2$ an $C^1$ gebunden ist bzw. $R^{2'}$ an $C^{1'}$ gebunden ist, womit zusammen mit $-C^1-O-O-C^{1'}-$eine substituierte oder unsubstituierte gesättigte oder teilweise ungesättigte carbocyclische Ring-Einheit mit 3 bis 14 C-Atomen gebildet wird, die zusätzlich zu den zumindest zwei O-Atomen gegebenenfalls 1 bis 4 weitere Heteroatome aufweist, wobei dieses carbocyclische Ring- oder Heteroring-System gegebenenfalls mit einem weiteren Ringsystem mit 4 bis 14 Ringatomen kondensiert ist;
oder funktionelle Derivate davon;
- mit der Maßgabe, daß mindestens zwei der Reste $R^1$, $R^2$ und $R^3$ bzw. mindestens zwei der Reste $R^{1'}$, $R^{2'}$ und $R^{3'}$ von H oder Methyl verschieden sind.

**2.** Polymerzusammensetzung nach Anspruch 1, die folgendes aufweist:

A) ein ungesättigtes Polymer und
B) eine Radikale bildende Verbindung,

- wobei die Polymerzusammensetzung und/oder das ungesättigte Polymer Kohlenstoff-Kohlenstoff-Doppelbindungen in einer Menge von mindestens 0,05, z.B. 0,1 oder mehr, stärker bevorzugt von 0,2 oder mehr und besonders bevorzugt von mehr als 0,37 Kohlenstoff-Kohlenstoff-Doppelbindungen/1000 Kohlenstoffatome aufweist; und
- wobei das Radikale bildende Mittel eine Verbindung der Formel (I) nach Anspruch 1 ist.

**3.** Verbindung der Formel (I) nach den Ansprüche 1 bis 2, wobei $R^2$ und $R^3$ zusammen mit dem Kohlenstoffatom ($C^1$), an das sie gebunden sind, eine gegebenenfalls substituierte carbocyclische Ring-Einheit mit 3 bis 12 C-Atomen im Ring oder eine gegebenenfalls substituierte Heteroring-Einheit mit 3 bis. 12 Ringatomen bilden, die 1 bis 6, vorzugsweise 1 bis 4 Heteroatome enthält, die aus O, N, P, S oder Si ausgewählt sind, und wobei das carbocyclische oder heterocyclische Ringsystem gegebenenfalls mit einem weiteren Ringsystem mit 4 bis 14 Ringatomen kondensiert ist, vorzugsweise bilden $R^2$ und $R^3$ zusammen mit dem Kohlenstoffatom ($C^1$) eine ($C_3$-$C_{12}$) carbocyclische Ring-Einheit; und
wobei $R^{2'}$ und $R^{3'}$ zusammen mit dem Kohlenstoffatom ($C^{1'}$), an das sie gebunden sind, eine gegebenenfalls substituierte carbocyclische Ring-Einheit mit 3 bis 12 C-Atomen im Ring oder eine gegebenenfalls substituierte Heteroring-Einheit mit 3 bis 12 Ringatomen bilden, die 1 bis 6, vorzugsweise 1 bis 4 Heteroatome enthält, die aus O, N, P, S oder Si auswählt sind, und wobei das carbocyclische oder heterocyclische Ringsystem gegebenenfalls mit einem weiteren Ringsystem mit 4 bis 14 Ringatomen kondensiert ist, vorzugsweise bilden $R^{2'}$ und $R^{3'}$ zusammen mit dem Kohlenstoffatom ($C^{1'}$) eine ($C_3$-$C_{12}$) carbocyclische Ring-Einheit; wobei vorzugsweise $R^2$ und $R^3$ zusammen mit dem Kohlenstoffatom ($C^1$), an das sie gebunden sind und/oder $R^{2'}$ und $R^{3'}$ zusammen mit dem Kohlenstoffatom ($C^{1'}$), an das sie gebunden sind, einen gegebenenfalls substituierten, gesättigten oder teilweise ungesättigten mono- oder bicyclischen ($C_4$-$C_{14}$) carbocylclischen Ring, stärker bevorzugt einen gegebenenfalls substituierten, gesättigten mono- cyclischen ($C_5$-$C_8$) carbocyclischen Ring bilden, wobei insbesondere
$R^2$ und $R^{2'}$ der gleiche Rest sind und $R^3$ und $R^{3'}$ der gleiche Rest sind.

**4.** Verbindung der Formel (I) nach einem der vorstehenden Ansprüche, wobei das Ringsystem, das von $R^{2'}$ und $R^{3'}$ zusammen mit dem Kohlenstoffatom ($C^{1'}$), an das sie gebunden sind, das gleiche Ringsystem ist, das von $R^2$ und $R^3$ zusammen mit dem Kohlenstoffatom ($C^1$), an das sie gebunden sind, gebildet wird;

- wobei $R^1$ und $R^{1'}$ jeweils für eine gegebenenfalls substituierte verzweigte oder geradkettige, vorzugsweise unsubstituierte geradkettige ($C_2$-$C_{30}$)-Alkylgruppe (vorzugsweise eine ($C_6$-$C_{30}$)-Alkylgruppe) oder Methyl stehen, wobei $R^1$ und $R^{1'}$ vorzugsweise gleich sind; wobei vorzugsweise
- $R^2$ und $R^3$ zusammen mit dem Kohlenstoffatom ($C^1$) an das sie gebunden sind, eine gegebenenfalls substituierte carbocyclische Ring-Einheit mit 3 bis 12 C-Atomen bilden, die gegebenenfalls mit einem weiteren Ringsystem mit 4 bis 14 Ringatomen kondensiert ist; und
- $R^{2'}$ und $R^{3'}$ zusammen mit dem Kohlenstoffatom ($C^{1'}$), an das sie gebunden sind, eine gegebenenfalls substituierte carbocyclische Ring-Einheit mit 3 bis 12 C-Atomen im Ring bilden, die gegebenenfalls mit einem weiteren Ringsystem mit 4 bis 14 Ringatomen kondensiert ist; und
- das Ringsystem, das von $R^{2'}$ und $R^{3'}$ zusammen mit dem Kohlenstoffatom ($C^{1'}$), an das sie gebunden sind, das gleiche wie das Ringsystem ist, das von $R^2$ und $R^3$ zusammen mit dem Kohlenstoffatom ($C^1$), an das sie gebunden sind, gebildet wird; und
- $R^1$ und $R^{1'}$ jeweils für eine gegebenenfalls substituierte, verzweigte oder geradkettige, vorzugsweise unsubstituierte geradkettige ($C_6$-$C_{30}$)-Alkylgruppe oder Methyl stehen.

**5.** Verbindung der Formel (I) nach den Ansprüchen 1 bis 4, wobei $R^1$, $R^2$, $R^3$, $R^{1'}$, $R^{2'}$ und $R^{3'}$ jeweils unabhängig voneinander eine gegebenenfalls substituierte mono- oder multicyclische ($C_5$-$C_{14}$)-Arylgruppe, eine gegebenenfalls substituierte mono- oder multicyclische ($C_5$-$C_{14}$)-Heteroarylgruppe; eine gegebenenfalls substituierte mono- oder multicyclische ($C_4$-$C_{14}$)-Cyclo-alkylgruppe; ein gegebenenfalls substituierter mono- oder multicyclischer ($C_4$-$C_{14}$)-heterocyclischer Rest; eine gegebenenfalls substituierte geradkettige oder verzweigte ($C_1$-$C_{50}$)-Alkylgruppe, vorzugsweise eine gegebenenfalls substituierte geradkettige ($C_1$-$C_{30}$)-Alkylgruppe; eine gegebenenfalls substituierte geradkettige oder verzweigte ($C_1$-$C_{50}$)-Alkenylgruppe oder gegebenenfalls substituierte geradkettige oder verzweigte ($C_1$-$C_{50}$)-Alkinylgruppe, vorzugsweise eine geradkettige ($C_1$-$C_{30}$)-Alkenyl- oder geradkettige ($C_1$-$C_{30}$)-Alkinylgruppe; eine gegebenenfalls substituierte geradkettige oder verzweigte ($C_1$-$C_{50}$)-Heteroalkylgruppe

mit 1 bis 4 Heteroatomen sind, die aus O, N, P, S oder Si ausgewählt sind; wobei vorzugsweise $R^2$, $R^{2'}$, $R^3$ und $R^{3'}$ unabhängig voneinander aus einer unsubstituierten geradkettigen ($C_1$-$C_{50}$)-Alkylgruppe, vorzugsweise einer ($C_1$-$C_{30}$)-Alkylgruppe, stärker bevorzugt einer ($C_1$-$C_{20}$)-Alkylgruppe, stärker bevorzugt einer $C_1$-$C_{12}$-Alkylgruppe, stärker bevorzugt aus Methyl oder ($C_6$-$C_{12}$)-Alkyl ausgewählt sind.

**6.** Verbindung der Formel (I) nach Anspruch 5, wobei

- $R^2$ und $R^{2'}$ gleich sind und jeweils Methyl darstellen; oder
- $R^2$ und $R^{2'}$ gleich sind und jeweils eine ($C_6$-$C_{30}$)-Alkylgruppe darstellen und/oder

wobei $R^3$ und $R^{3'}$ gleich sind und jeweils eine ($C_6$-$C_{30}$)-Alkylgruppe darstellen.

**7.** Verbindung der Formel (I) nach einem der vorstehenden Ansprüche, die aus folgendem ausgewählt ist:

- $R^1$ und $R^{1'}$ sind gleich oder verschieden, vorzugsweise gleich, und stehen jeweils für einen gegebenenfalls substituierten, gesättigten oder teilweise ungesättigten cyclischen Kohlenwasserstoffrest mit 5 bis 14 Ringatomen, der gegebenenfalls 1 bis 4 Heteroatome im Ring enthält, die aus N, O, P, S oder Si ausgewählt sind, oder eine gegebenenfalls substituierte mono- oder multicyclische ($C_5$-$C_{14}$)-Arylgruppe, vorzugsweise eine unsubstituierte monocyclische ($C_5$-$C_7$)-Aryl; oder
- $R^1$ und $R^{1'}$ sind gleich oder verschieden, vorzugsweise gleich, und stehen jeweils für eine gegebenenfalls substituierte, verzweigte oder geradkettige, vorzugsweise unsubstituierte geradkettige ($C_6$-$C_{30}$)-Alkylgruppe oder Methyl.

**8.** Verbindung der Formel (I) nach einem der vorstehenden Ansprüche, wobei $R^1$ und $R^{1'}$ gleich sind und jeweils für Methyl stehen; und
R2 und $R^3$ zusammen mit dem $C^1$-Atom, an das sie gebunden sind, einen gegebenenfalls substituierten, gesättigten oder teilweise ungesättigten mono- oder bicyclischen ($C_4$-$C_{14}$) carbocyclischen Ring, vorzugsweise einen unsubstituierten gesättigten monocyclischen ($C_5$-$C_8$) carbocyclischen Ring bilden; und
$R^{2'}$ und $R^{3'}$ zusammen mit dem Kohlenstoffatom ($C^{1'}$), an das sie gebunden sind, einen gegebenenfalls substituierten, gesättigten oder teilweise ungesättigten mono- oder bicyclischen ($C_4$-$C_{14}$) carbocyclischen Ring, vorzugsweise einen unsubstituierten gesättigten monocyclischen ($C_5$-$C_8$) carbocyclischen Ring bilden; wobei das Ringsystem, das von $R^2$ und $R^3$ zusammen mit $C^1$ gebildet wird, einem Ringsystem vorzugsweise identisch ist, das von $R^{2'}$ und $R^{3'}$ zusammen mit $C^{1'}$ gebildet wird.

**9.** Verbindung nach Anspruch 8 der Formel (II)

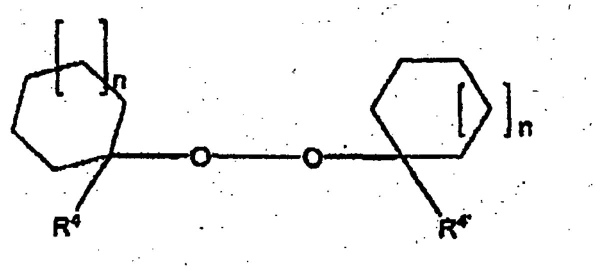

(II)

worin n gleich 0 bis 3 ist und $R^4$ und $R^{4'}$ jeweils unabhängig voneinander für eine geradkettige Alkylgruppe mit 1 bis 30 Kohlenstoffatomen, vorzugsweise Methyl, oder eine geradkettige Alkylgruppe mit 6 bis 20, vorzugsweise 6 bis 12 Kohlenstoffatomen, stärker bevorzugt Methyl stehen, und wobei ein Ringsystem oder beide unabhängig voneinander unsubstituiert oder gegebenenfalls mit 1 bis 4 Substituenten substituiert ist bzw. sind.

**10.** Verbindung nach einem der vorstehenden Ansprüche 4 bis 9, wobei $R^1$ und $R^{1'}$ gleich sind und für eine gegebenenfalls substituierte, vorzugsweise unsubstituierte monocyclische ($C_5$-$C_7$)-Arylgruppe stehen;
$R^2$ und $R^{2'}$ gleich und beide Methyl sind; und
$R^3$ und $R^{3'}$ gleich sind und beide eine gegebenenfalls substituierte verzweigte oder geradkettige ($C_6$-$C_{50}$)-Alkylgruppe, stärker bevorzugt eine unsubstituierte geradkettige ($C_6$-$C_{30}$)-Alkylgruppe, wie eine ($C_6$-$C_{20}$)-Alkylgruppe, sind.

**11.** Verbindung nach Anspruch 10 der Formel (III)

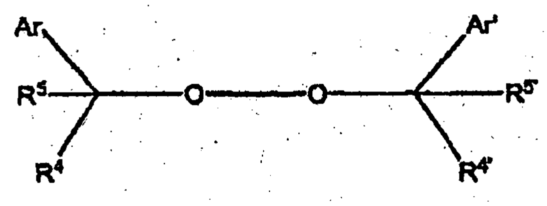

worin Ar und Ar' unabhängig voneinander für eine Phenyl-, Benzyl- oder Naphthylgruppe stehen, die gegebenenfalls mit 1 bis 4 Substituenten substituiert ist,
$R^4$ und $R^{4'}$ jeweils Methyl sind, und
$R^5$ und $R^{5'}$ jeweils unabhängig voneinander für eine geradkettige Alkylgruppe mit 6 bis 30 Kohlenstoffatomen, vorzugsweise 6 bis 20, stärker bevorzugt 6 bits 12 Kohlenstoffatomen stehen.

**12.** Verbindung der Formel (I) nach einem der vorstehenden Ansprüche, wobei die wahlfreien Substituenten jeweils unabhängig voneinander ausgewählt sind aus -OH, $-NR_2$, wobei R jeweils unabhängig voneinander H oder eine $(C_1-C_{12})$-Alkylgruppe ist, COR", wobei R" unter anderem H, eine $(C_1-C_{12})$-Alkylgruppe oder $-NR_2$ ist, wobei R jeweils wie für $-NR_2$ definiert ist, COOR", wobei R wie für -COR" definiert ist; Halogen, wie -F, -Cl oder -I; oder Alkoxy, einem gesättigten oder teilweise ungesättigtem Kohlenwasserstoffrest, der gegebenenfalls eine funktionelle Gruppe aufweist; oder einem aromatischen Kohlenwasserstoffrest, der gegebenenfalls eine funktionelle Gruppe aufweist.

**13.** Verbindung nach Anspruch 1 der Formel (V)

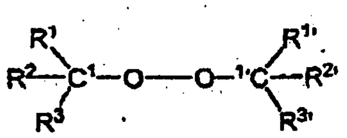

(V)

wobei die Verbindungen aus irgendeiner der Möglichkeiten (i) bis (iii) ausgewählt sind:

(i) - $R^1$ und $R^{1'}$ sind jeweils unabhängig voneinander H, ein substituierter oder substituierter gesättigter oder teilweise ungesättigter Kohlenwasserstoffrest;

- wobei jeder der substituierten öder unsubstituierten gesättigten oder teilweise ungesättigten Köhlenwasserstöffreste gegebenenfalls ein oder mehr Heteroatome aufweist;
- wobei der substituierte oder unsubstituierte gesättigte oder teilweise ungesättigte Kohlenwasserstoffrest (i) geradkettige oder verzweigte gesättigte oder teilweise ungesättigte Kohlenwasserstoffreste, (ii) geradkettige oder verzweigte gesättigte oder teilweise ungesättigte Kohlenwasserstoffreste, die einen gesättigten oder teilweise ungesättigten cyclischen Kohlenwasserstoffrest aufweisen, und (iii) gesättigte oder teilweise ungesättigte cyclische Kohlenwasserstoffreste einschließt;
- wobei jeder gesättigten oder teilweise ungesättigten cyclischen Kohlenwasserstoffreste unabhängig voneinander ein monocyclisches oder multicyclisches Ringsystem ist; und
- wobei der substituierte gesättigte oder teilweise ungesättigte Kohlenwasserstoffrest unabhängig 1 bis 4 Substituenten aufweist, die ausgewählt sind aus einer funktionellen Gruppe, einem gesättigten öder teilweise ungesättigten Kohlenwasserstoffrest, der gegebenenfalls eine funktionelle Gruppe aufweist, oder einem aromatischen Kohlenwasserstoffrest, der gegebenenfalls eine funktionelle Gruppe aufweist; und
- $R^2$, $R^{2'}$-, R3 und $R^{3'}$ jeweils unabhängig voneinander wie vorstehend für $R^1$ und $R^{1'}$ angegeben sind;

oder
(ii) - $R^1$ und $R^{1'}$ jeweils unabhängig voneinander eine gegebenenfalls substituierte, vorzugsweise unsubstituierte, monocyclische $(C_5-C_7)$Arylgruppe, vorzugsweise Phenyl, sind,

- wobei die substituierte monocyclische $(C_5-C_7)$Arylgruppe unabhängig 1 bis 4 Substituenten aufweist, die ausgewählt sind aus einer funktionellen Gruppe, einem gesättigten oder teilweise ungesättigten Kohlenwasserstoffrest, der gegebenenfalls eine funktionelle Gruppe aufweist, oder einem aromatischen Kohlenwasserstoffrest, der gegebenenfalls eine funktionelle Gruppe aufweist; und

$R^2$ und $R^{2'}$ gleich und jeweils Methyl sind; und $R^3$ und $R^{3'}$ jeweils unabhängig voneinander H, ein substituierter oder unsubstituierter gesättigter oder teilweise ungesättigter Kohlenwasserstoffrest wie vorstehend unter (i) für $R^1$ und $R^{1'}$ angegeben sind; oder
(iii) - $R^1$ und $R^{1'}$ jeweils unabhängig voneinander H, ein substituierter oder unsubstituierter gesättigter oder teilweise ungesättigter Kohlenwasserstoffrest wie vorstehend unter (i) für $R^1$ und $R^{1'}$ angegeben sind; und

- $R^2$ und $R^3$ zusammen mit dem Kohlenstoffatom ($C^1$), an das sie gebunden sind, eine unsubstituierte oder substituierte gesättigte oder teilweise ungesättigte carbocyclicsche Ring-Einheit mit 3 bis 14 C-Atomen, vorzugsweise 5 bis 12 C-Atomen, oder eine unsubstituierte oder substituierte gesättigte oder teilweise ungesättigte Heteroring-Einheit mit 3 bis 14 Ringatomen bilden, die 1 bis 6, vorzugsweise 1 bis 4 Heteroatome aufweist, die aus O, N, P, S oder Si ausgewählt sind;

  - wobei der carbocyclische Ring oder Heteroring gegebenenfalls mit einem weiteren gegebenenfalls substituierten Ringsystem kondensiert ist, das 4 bis 14 Ringatome hat; und
  - wobei das substituierte carbocyclische Ring- oder Heteroring-System 1 bis 4 Substituenten aufweist, die unabhängig voneinander aus einer funktionellen Gruppe oder einem gesättigten oder teilweise ungesättigten Kohlenwasserstoffrest ausgewählt sind, der eine funktionelle Gruppe aufweist; und

- $R^{2'}$ und $R^{3'}$ zusammen mit dem Kohlenstoffatom ($C^{1'}$), an das sie gebunden sind, eine unsubstituierte oder substituierte gesättigte oder teilweise ungesättigte carbocyclische Ring-Einheit mit 3 bis 14 C-Atomen, vorzugsweise 5 bis 12 C-Atomen, eine unsubstituierte oder substituierte gesättigte oder teilweise ungesättigte Heteroring-Einheit mit 3 bis 14 Ringatomen bilden, die 1 bis 6, vorzugsweise 1 bis 4 Heteroatome aufweist, die aus O, N, P, S oder Si ausgewählt sind;

  - wobei das carbocyclisches Ring- oder Heteroring-System gegebenenfalls mit einem weiteren gegebenenfalls substituierten Ringsystem mit 4 bis 14 Ringatomen kondensiert ist; und
  - wobei das substituierte carbocyclische Ring- oder Heteroring-System 1 bis 4 Substituenten aufweist, die unabhängig voneinander aus einer funktionellen Gruppe oder einem gesättigten oder teilweise ungesättigten Kohlenwasserstoffrest ausgewählt sind, der gegebenenfalls eine funktionelle Gruppe aufweist;

- mit der Maßgabe für die Möglichkeiten (i) bis (iii), das mindestens zwei der Reste $R^1$, $R^2$ und $R^3$ bzw. mindestens zwei der Reste $R^{1'}$, $R^{2'}$ und $R^{3'}$ von H öder Methyl verschieden sind.

**14.** Verbindung nach Anspruch 1 oder 2, die aus irgendeiner der folgenden ausgewählt ist:

- Di(1-methylcyclopentyl)peroxid
- Di-(1-methyl-1-phenylundecyl)peroxid
- Di-(1-methyl-1-phenylheptyl)peroxid oder
- Di(1-methyl-cyclohexyl)peroxid.

**15.** Verbindung nach einem der vorstehenden Ansprüche, die in ihrer Struktur ein oder mehrere Einheiten aufweist, die sich in einem Radikalerzeugungsschritt zu einem Zersetzungsprodukt zersetzen lassen,

- wobei die eine oder mehreren zersetzbaren Einheiten zu einem $CH_4$-Gehalt von weniger als 300 ppm (Gewicht), vorzugsweise von weniger als 200 ppm (Gewicht), vorzugsweise von weniger als 100 ppm (Gewicht), stärker bevorzugt von 0 bis 50 ppm (Gewicht) führen, und zwar nach einem Verfahren bestimmt, wie es in dieser Beschreibung unter "GC-Analyseprotokoll" beschrieben ist; oder
- eine Verbindung ohne irgendeine derartige Einheit, die sich in $CH_4$ als Zersetzungsprodukt zersetzen läßt, oder irgendein Gemisch davon.

**16.** Zusammensetzung nach einem der vorstehenden Ansprüche, wobei das ungesättigte Polymer ein ungesättigtes LDPE-Homopolymer oder LDPE-Copolymer mit einem oder mehreren mehrfach ungesättigten Comonomeren, vorzugsweise einem oder mehreren Dienen, ist, insbesondere eine in Form von (1) Polymerpulver, (2) Polymergranulat oder (3) einem Gemisch aus einer Polymerschmelze, wobei das (1) Polymerpulver oder vorzugsweise (2) Polymergranulat wahlfrei in einem Behälter enthalten ist.

**17.** Modifizierte Polymerzusammensetzung, wobei die Polymerzusammensetzung nach einem der vorstehenden Ansprüche durch Einleitung einer Radikalreaktion in der.Polymerzusammensetzung vernetzt ist.

**18.** Vernetzbares Kabel, das einen Leiter aufweist, der von einer mehreren Schichten umgeben wird, z.B. einer Isolationsschicht, einer Ummantelungsschicht und/oder einer halbleitenden Schicht, umfassend eine Polymerzusammensetzung nach den Ansprüchen 1 bis 16, oder vernetztes Kabel, bei dem die Polymerzusammensetzung durch Einleitung einer Radikalreaktion in der Polymerzusammensetzung vernetzt worden ist, vorzugsweise

- ein Niederspannungskabel, das einen Leiter umfaßt, der von einer Isolationsschicht und gegebenenfalls einer Ummantelungsschicht umgeben wird, wobei die Isolationsschicht eine Polymerzusammensetzung nach den Ansprüche 1 bis 16 umfaßt; oder
- ein Stromkabel, das einen elektrischen Leiter aufweist, der von einer oder mehreren Schichten umgeben wird, umfassend in dieser Reihenfolge zumindest eine innere halbleitende Schicht, eine Isolationsschicht und eine äußere halbleitende Schicht, und das gegebenenfalls von einer Ummantelungsschicht umschlossen wird, wobei zumindest eine dieser Schichten eine Polymerzusammensetzung nach den Ansprüchen 1 bis 16 umfaßt.

## Revendications

**1.** Composition de polymère comprenant

A) un polymère insaturé, et
B) un composé générateur de radicaux libres

- dans laquelle le composé générateur de radicaux libres est un composé de formule (I)

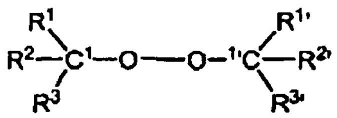

(I)

dans laquelle
- $R^1$et $R^{1'}$sont chacun indépendamment H, un hydrocarbyle substitué ou non, saturé ou partiellement insaturé ; ou un hydrocarbyle aromatique substitué ou non ;
- chacun desdits hydrocarbyle substitué ou non, saturé ou partiellement insaturé, ou hydrocarbyle aromatique comprenant éventuellement un ou plusieurs hétéroatomes ; et
- ledit hydrocarbyle substitué, saturé ou partiellement insaturé, ou hydrocarbyle aromatique substitué comprenant indépendamment de 1 à 4 substituants choisis parmi un groupe fonctionnel, ou un hydrocarbyle saturé ou partiellement insaturé portant éventuellement un groupe fonctionnel ; ou un hydrocarbyle aromatique portant éventuellement un groupe fonctionnel ;
- $R^2$, $R^{2'}$, $R^3$ et $R^{3'}$ sont chacun indépendamment H, un hydrocarbyle substitué ou non, saturé ou partiellement insaturé ; ou un hydrocarbyle aromatique substitué ou non ;
- chacun desdits hydrocarbyle substitué ou non, saturé ou partiellement insaturé, ou hydrocarbyle aromatique comprenant éventuellement un ou plusieurs hétéroatomes ; et
- ledit hydrocarbyle substitué, saturé ou partiellement insaturé, ou hydrocarbyle aromatique substitué comprenant indépendamment de 1 à 4 substituants choisis parmi un groupe fonctionnel, un hydrocarbyle saturé ou partiellement insaturé portant éventuellement un groupe fonctionnel ; ou un hydrocarbyle aromatique portant éventuellement un groupe fonctionnel ; ou
- $R^2$ et $R^3$, avec l'atome de carbone ($C^1$) auquel ils sont liés, forment un fragment du type cycle carbocyclique non substitué ou substitué, saturé ou partiellement insaturé ayant de 3 à 14 atomes C ; un fragment du type hétérocycle non substitué ou substitué, saturé ou partiellement insaturé ayant de 3 à 14 atomes dans le cycle comprenant de 1 à 6 hétéroatomes choisis parmi O, N, P, S ou Si ; ou un fragment du type cycle aromatique non substitué ou substitué ayant de 3 à 14 atomes C comprenant éventuellement de 1 à 4 hétéroatomes ;

- ledit système de cycle carbocyclique, hétérocyclique ou aromatique étant éventuellement condensé avec un autre système de cycle ayant de 4 à 14 atomes dans le cycle ; et
- ledit système de cycle carbocyclique, hétérocyclique ou aromatique substitué comprenant de 1 à 4 substituants choisis indépendamment parmi un groupe fonctionnel, un hydrocarbyle saturé ou partiellement insaturé portant éventuellement un groupe fonctionnel ; ou un hydrocarbyle aromatique portant éventuellement un groupe fonctionnel ; ou
- $R^{2'}$ et $R^{3'}$, avec l'atome de carbone ($C^{1'}$) auquel ils sont liés, forment un fragment du type cycle carbocyclique non substitué ou substitué, saturé ou partiellement insaturé ayant de 3 à 14 atomes C ; un fragment du type hétérocycle non substitué ou substitué, saturé ou partiellement insaturé ayant de 3 à 14 atomes dans le cycle comprenant de 1 à 6 hétéroatomes choisis parmi O, N, P, S ou Si ; ou un fragment du type cycle aromatique non substitué ou substitué ayant de 3 à 14 atomes C comprenant éventuellement de 1 à 4 hétéroatomes ;
- ledit système de cycle carbocyclique, hétérocyclique ou aromatique étant éventuellement condensé avec un autre système de cycle ayant de 4 à 14 atomes dans le cycle ; et
- ledit système de cycle carbocyclique, hétérocyclique ou aromatique substitué comprenant de 1 à 4 substituants choisis indépendamment parmi un groupe fonctionnel, un hydrocarbyle saturé ou partiellement insaturé portant éventuellement un groupe fonctionnel ; ou un hydrocarbyle aromatique portant éventuellement un groupe fonctionnel ; ou
- $R^2$ et $R^{2'}$ forment ensemble un hydrocarbyle bivalent substitué ou non, saturé ou partiellement insaturé contenant éventuellement de 1 à 4 hétéroatomes, $R^2$ étant lié à $C^1$ et $R^{2'}$ à $C^{1'}$, respectivement, pour former avec $-C^1-O-O-C^{1'}-$ un fragment du type cycle carbocyclique substitué ou non, saturé ou partiellement insaturé ayant de 3 à 14 atomes C comprenant, éventuellement, en plus desdits au moins deux atomes O, de 1 à 4 hétéroatomes supplémentaires ; ledit système de cycle carbocylique ou hétérocyclique étant éventuellement condensé avec un autre système de cycle ayant de 4 à 14 atomes dans le cycle ;

ou des dérivés fonctionnels de celui-ci ;

- à condition qu'au moins deux des $R^1$, $R^2$ et $R^3$, et au moins deux des $R^{1'}$, $R^{2'}$ et $R^{3'}$, respectivement, soient autres que H ou méthyle.

**2.** Composition de polymère selon la revendication 1 comprenant

A) un polymère insaturé, et
B) un composé générateur de radicaux libres

- la composition de polymère et/ou le polymère insaturé contenant des doubles liaisons carbone-carbone en une quantité d'au moins 0,05, par exemple, 0,1 ou plus, plus préférablement, 0,2 ou plus, et de manière préférée entre toutes, supérieure à 0,37 doubles liaisons carbone-carbone/ 1000 atomes de carbone, et
- l'agent générateur de radicaux libres étant un composé de formule (I) selon la revendication 1.

**3.** Composé de formule (I) selon les revendications 1 à 2,

- dans lequel $R^2$ et $R^3$, avec l'atome de carbone ($C^1$) auquel ils sont liés, forment un fragment du type cycle carbocyclique éventuellement substitué, ayant de 3 à 12 atomes C dans le cycle ou un fragment du type hétérocycle éventuellement substitué, ayant de 3 à 12 atomes dans le cycle contenant de 1 à 6, de préférence, de 1 à 4, hétéroatomes choisis parmi O, N, P, S ou Si, et dans lequel ledit système de cycle carbocyclique ou hétérocyclique est éventuellement condensé avec un autre système de cycle ayant de 4 à 14 atomes dans le cycle, de préférence, $R^2$ et $R^3$ formant, avec l'atome de carbone ($C^1$), un fragment du type cycle carbocyclique (C3-C12) ; et
- dans lequel $R^{2'}$ et $R^{3'}$, avec l'atome de carbone ($C^{1'}$) auquel ils sont liés, forment un fragment du type cycle carbocyclique éventuellement substitué, ayant de 3 à 12 atomes C dans le cycle ou un fragment du type hétérocycle éventuellement substitué, ayant de 3 à 12 atomes dans le cycle contenant de 1 à 6, de préférence, de 1 à 4, hétéroatomes choisis parmi O, N, P, S ou Si, et dans lequel ledit système de cycle carbocyclique ou hétérocyclique est éventuellement condensé avec un autre système de cycle ayant de 4 à 14 atomes dans le cycle, de préférence, $R^{2'}$ et $R^{3'}$ formant, avec l'atome de carbone ($C^{1'}$), un fragment du type cycle carbocyclique (C3-C12) ; de préférence,
- dans lequel $R^2$ et $R^3$, avec l'atome de carbone ($C^1$) auquel ils sont liés, et/ou $R^{2'}$ et $R^{3'}$, avec l'atome de carbone ($C^{1'}$) auquel ils sont liés, forment un cycle carbocyclique mono- ou bicyclique (C4-C14) éventuellement

substitué, saturé ou partiellement insaturé, plus préférablement, un cycle carbocyclique monocyclique (C5-C8) éventuellement substitué, saturé, et notamment
- dans lequel $R^2$ et $R^{2'}$ représentent le même radical, et $R^3$ et $R^{3'}$ représentent le même radical.

**4.** Composé de formule (I) selon l'une quelconque des revendications précédentes, dans lequel le système de cycle formé par $R^{2'}$ et $R^{3'}$, avec l'atome de carbone ($C^{1'}$) auquel ils sont liés est identique au système de cycle formé par $R^2$ et $R^3$, avec l'atome de carbone ($C^1$) auquel ils sont liés ; et

- dans lequel $R^1$ et $R^{1'}$ représentent chacun un alkyle (C2-C30) à chaîne ramifiée ou droite, éventuellement substitué, de préférence, non substitué (de préférence, un alkyle (C6-C30) ou un méthyle, de préférence, dans lequel $R^1$ et $R^{1'}$ sont identiques ; de préférence,
- dans lequel $R^2$ et $R^3$, avec l'atome de carbone ($C^1$) auquel ils sont liés, forment un fragment du type cycle carbocyclique éventuellement substitué ayant de 3 à 12 atomes C dans le cycle qui est éventuellement condensé avec un autre système de cycle ayant de 4 à 14 atomes dans le cycle, et
- dans lequel $R^{2'}$ et $R^{3'}$, avec l'atome de carbone ($C^{1'}$) auquel ils sont liés, forment un fragment du type cycle carbocyclique éventuellement substitué ayant de 3 à 12 atomes C dans le cycle qui est éventuellement condensé avec un autre système de cycle ayant de 4 à 14 atomes dans le cycle, et
- dans lequel le système de cycle formé par $R^{2'}$ et $R^{3'}$, avec l'atome de carbone ($C^{1'}$) auquel ils sont liés, est identique au système de cycle formé par $R^2$ et $R^3$, avec l'atome de carbone ($C^1$) auquel ils sont liés, et
- dans lequel $R^1$ et $R^{1'}$ représentent chacun un alkyle (C6-C30) à chaîne ramifiée ou droite, éventuellement substitué, de préférence, non substitué ou un méthyle.

**5.** Composé de formule (I) selon les revendications 1 à 4, dans lequel $R^1$, $R^2$, $R^3$, $R^{1'}$, $R^{2'}$ et $R^{3'}$ sont chacun indépendamment un aryle mono- ou multicyclique (C5-C14) éventuellement substitué ; un hétéroaryle mono- ou multicyclique (C5-C14) éventuellement substitué; un cycloalkyle mono- ou multicyclique (C4-C14) éventuellement substitué ; un hétérocyclyle mono- ou multicyclique (C4-C14) éventuellement substitué ; un alkyle (C1-C50) à chaîne droite ou ramifiée éventuellement substitué, de préférence, un alkyle (C1-C30) à chaîne droite éventuellement substitué ; un alcényle (C 1-C50) à chaîne droite ou ramifiée éventuellement substitué ou un alcynyle (C1-C50) à chaîne droite ou ramifiée éventuellement substitué, de préférence, un alcényle (C1-C30) à chaîne droite ou un alcynyle (C 1-C30) à chaîne droite ; un hétéroalkyle (C1-C50) à chaîne droite ou ramifiée éventuellement substitué comprenant de 1 à 4 hétéroatomes choisis parmi O, N, P, S ou Si ; de préférence,
dans lequel $R^2$, $R^{2'}$, $R^3$ et $R^{3'}$ sont chacun indépendamment choisis parmi un alkyle (C1-C50) à chaîne droite non substitué, de préférence, un alkyle (C1-C30), plus préférablement, un alkyle (C1-C20), plus préférablement, un alkyle (C1-C12), et mieux encore, un méthyle ou un alkyle (C6-C 12).

**6.** Composé de formule (I) selon la revendication 5, dans lequel

- $R^2$ et $R^{2'}$ sont identiques et chacun représente un méthyle ; ou
- $R^2$ et $R^{2'}$ sont identiques et chacun représente un alkyle (C6-C30) et/ou

dans lequel $R^3$ et $R^{3'}$ sont identiques et chacun représente un alkyle (C6-C30).

**7.** Composé de formule (I) selon l'une quelconque des revendications précédentes, dans lequel

- $R^1$ et $R^{1'}$ sont identiques ou différents, de préférence, identiques, et chacun représente un hydrocarbyle cyclique éventuellement substitué, saturé ou partiellement insaturé, ayant de 5 à 14 atomes dans le cycle contenant éventuellement de 1 à 4 hétéroatomes choisis parmi N, O, P, S ou Si ; ou un aryle mono- ou multicyclique (C5-C14) éventuellement substitué, de préférence, un aryle monocyclique (C5-C7) non substitué ; ou
- $R^1$ et $R^{1'}$ sont identiques ou différents, de préférence, identiques, et chacun représente un alkyle (C6-C30) éventuellement substitué, à chaîne droite ou ramifiée, de préférence, non substitué et à chaîne droite ou un méthyle.

**8.** Composé de formule (I) selon l'une quelconque des revendications précédentes, dans lequel $R^1$ et $R^{1'}$ sont identiques et chacun représente un méthyle ; et
$R^2$ et $R^3$, avec l'atome de carbone $C^1$ auquel ils sont liés, forment un cycle carbocyclique mono- ou bicyclique (C4-C 14) éventuellement substitué, saturé ou partiellement insaturé, de préférence, un cycle carbocyclique monocyclique (C5-C8) non substitué, saturé ;
et $R^{2'}$ et $R^{3'}$, avec l'atome de carbone $C^1$ auquel ils sont liés, forment un cycle carbocyclique mono- ou bicyclique

(C4-C14) éventuellement substitué, saturé ou partiellement insaturé, de préférence, un cycle carbocyclique monocyclique (C5-C8) non substitué, saturé ; de façon que le système de cycle formé par $R^2$ et $R^3$ avec $C^1$ soit, de préférence, identique au système de cycle formé par $R^{2'}$ et $R^{3'}$, avec $C^{1'}$.

**9.** Composé selon la revendication 8 de formule (II)

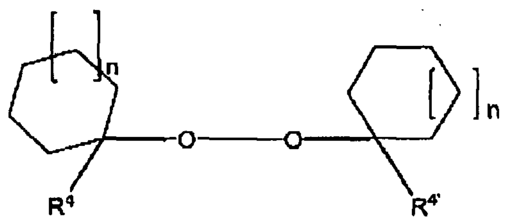

(II)

dans laquelle n vaut de 0 à 3, et $R^4$ et $R^{4'}$ représentent chacun indépendamment un groupe alkyle à chaîne droite ayant de 1 à 30 atomes de carbone, de préférence, un méthyle ou un groupe alkyle à chaîne droite ayant de 6 à 20, de préférence, de 6 à 12 atomes de carbone, et dans laquelle un ou les deux systèmes de cycles sont indépendamment non substitués ou éventuellement substitués par de 1 à 4 substituants.

**10.** Composé selon l'une quelconque des revendications 4 à 9 précédentes, dans lequel $R^1$ et $R^{1'}$ sont identiques et représentent un aryle monocyclique (C5-C7) éventuellement substitué, de préférence, non substitué ;
$R^2$ et $R^{2'}$ sont identiques et tous les deux méthyle ; et
$R^3$ et $R^{3'}$ sont identiques et tous les deux un alkyle (C6-C50) à chaîne ramifiée ou droite, éventuellement substitué, plus préférablement, un alkyle (C6-C30) à chaîne droite, non substitué, tel qu'un alkyle (C6-C20).

**11.** Composé selon la revendication 10 de formule (III)

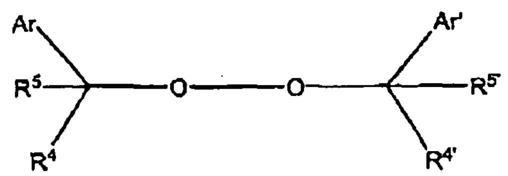

dans laquelle Ar et Ar' représentent indépendamment un groupe phényle, benzyle ou naphtyle éventuellement substitué par de 1 à 4 substituants,
$R^4$ et $R^{4'}$ sont chacun un méthyle ; et
$R^5$ et $R^{5'}$ représentent chacun indépendamment un groupe alkyle à chaîne droite ayant de 6 à 30 atomes de carbone, de préférence, de 6 à 20, plus préférablement, de 6 à 12, atomes de carbone.

**12.** Composé de formule (I) selon l'une quelconque des revendications précédentes, dans lequel lesdits éventuels substituants sont chacun indépendamment choisis parmi -OH, $-NR_2$, où chaque R est indépendamment H ou un alkyle (C1-C12), COR", où R" est, par exemple, H, un alkyle (C1-C12) ou $-NR_2$, où R est tel que défini pour $-NR_2$, COOR", où R est tel que défini pour -COR" ; un atome d'halogène, tel que -F, -Cl ou -I ; ou un alcoxy, un hydrocarbyle saturé ou partiellement insaturé portant éventuellement un groupe fonctionnel ; ou un hydrocarbyle portant éventuellement un groupe fonctionnel.

**13.** Composé selon la revendication 1 de formule (V)

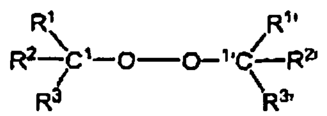

(V)

dans laquelle les composés sont choisis parmi l'une quelconque des alternatives (i) à (iii) suivantes :

(i) - $R^1$ et $R^{1'}$ sont chacun indépendamment H, un hydrocarbyle substitué ou non, saturé ou partiellement insaturé ;

- chacun desdits hydrocarbyles substitués ou non, saturés ou partiellement insaturés comprenant éventuellement un ou plusieurs hétéroatomes ;
- lesdits hydrocarbyles substitués ou non, saturés ou partiellement insaturés comprenant (i) les hydrocarbyles à chaîne droite ou ramifiée saturés ou partiellement insaturés, (ii) les hydrocarbyles à chaîne droite ou ramifiée saturés ou partiellement insaturés qui portent un hydrocarbyle cyclique saturé ou partiellement insaturé et (iii) les hydrocarbyles cycliques saturés ou partiellement insaturés ;
- chacun desdits hydrocarbyles cycliques saturés ou partiellement insaturés étant indépendamment un système de cycle monocyclique ou multicyclique ; et
- lesdits hydrocarbyles substitués, saturés ou partiellement insaturés comprenant indépendamment de 1 à 4 substituants choisis parmi un groupe fonctionnel, un hydrocarbyle saturé ou partiellement insaturé portant éventuellement un groupe fonctionnel ou un hydrocarbyle aromatique portant éventuellement un groupe fonctionnel ; et
- $R^2$, $R^{2'}$, $R^3$ et $R^{3'}$ sont chacun indépendamment tels que définis ci-dessus pour $R^1$ et $R^{1'}$ ; ou

(ii) - $R^1$ et $R^{1'}$ sont chacun indépendamment un aryle monocyclique (C5-C7) éventuellement substitué, de préférence, non substitué, de préférence, un phényle,

l edit aryle monocyclique (C5-C7) substitué comprenant indépendamment de 1 à 4 substituants choisis parmi un groupe fonctionnel, un hydrocarbyle saturé ou partiellement insaturé portant éventuellement un groupe fonctionnel ou un hydrocarbyle aromatique portant éventuellement un groupe fonctionnel ; et

$R^2$ et $R^{2'}$ sont identiques et sont tous deux méthyle ; et
$R^3$ et $R^{3'}$ sont chacun indépendamment H, un hydrocarbyle substitué ou non, saturé ou partiellement insaturé tel que défini ci-dessus en (i) pour $R^1$ et $R^{1'}$ ; ou
(iii) - $R^1$ et $R^{1'}$ sont chacun indépendamment H, un hydrocarbyle substitué ou non, saturé ou partiellement insaturé tel que défini ci-dessus en (i) pour $R^1$ et $R^{1'}$ ; et

- $R^2$ et $R^3$, avec l'atome de carbone ($C^1$) auquel ils sont liés, forment un fragment du type cycle carbocyclique non substitué ou substitué, saturé ou partiellement insaturé ayant de 3 à 14 atomes C, de préférence, de 5 à 12 atomes C ; ou un fragment du type hétérocycle non substitué ou substitué, saturé ou partiellement insaturé ayant de 3 à 14 atomes dans le cycle comprenant de 1 à 6, de préférence, de 1 à 4 hétéroatomes, choisis parmi O, N, P, S ou Si ;
- ledit cycle carbocyclique ou hétérocycle étant éventuellement condensé avec un autre système de cycle éventuellement substitué ayant de 4 à 14 atomes dans le cycle ; et
- ledit système de cycle carbocyclique ou hétérocyclique substitué comprenant de 1 à 4 substituants choisis indépendamment parmi un groupe fonctionnel, ou un hydrocarbyle saturé ou partiellement insaturé portant éventuellement un groupe fonctionnel ; et
- $R^{2'}$ et $R^{3'}$, avec l'atome de carbone ($C^{1'}$) auquel ils sont liés, forment un fragment du type cycle carbocyclique non substitué ou substitué, saturé ou partiellement insaturé ayant de 3 à 14 atomes C, de préférence, de 5 à 12 atomes C ; ou un fragment du type hétérocycle non substitué ou substitué, saturé ou partiellement insaturé ayant de 3 à 14 atomes dans le cycle comprenant de 1 à 6, de préférence, de 1 à 4, hétéroatomes, choisis parmi O, N, P, S ou Si ;
- ledit système de cycle carbocyclique ou hétérocyclique étant éventuellement condensé avec un autre système de cycle éventuellement substitué ayant de 4 à 14 atomes dans le cycle ; et
- ledit système de cycle carbocyclique ou hétérocyclique substitué comprenant de 1 à 4 substituants choisis indépendamment parmi un groupe fonctionnel, ou un hydrocarbyle saturé ou partiellement insaturé portant éventuellement un groupe fonctionnel ;
- à condition que, pour les alternatives (i) à (iii), au moins deux des $R^1$, $R^2$ et $R^3$, et au moins deux des $R^{1'}$, $R^{2'}$ et $R^{3'}$, respectivement, soient autres que H ou méthyle.

**14.** Composé selon les revendications 1 à 2 qui est choisi parmi l'un quelconque des composés suivants

- peroxyde de di-(1-méthylcyclopentyle)

- peroxyde de di-(1-méthyl-1-phénylundécyle)
- peroxyde de di-(1-méthyl-1-phénylheptyle) ou
- peroxyde de di-(-méthyl-cyclohexyle).

**15.** Composé selon l'une quelconque des revendications précédentes qui porte un ou plusieurs fragments dans sa structure qui peuvent être décomposés en un produit de décomposition dans une étape de génération de radicaux libres

- dans lequel ledit ou lesdits fragments pouvant être décomposés induisent une teneur en $CH_4$, déterminée selon un procédé décrit dans la description sous le titre « Protocole d'analyse GC », qui est inférieure à 300 ppm (en poids), de préférence, inférieure à 200 ppm (en poids), voire, inférieure à 100 ppm (en poids), plus préférablement, de 0 à 50 ppm (en poids) ; ou

composé dépourvu de fragment qui peut être décomposé en $CH_4$ à titre dudit produit de décomposition ; ou mélange de ceux-ci.

**16.** Composition selon l'une quelconque des revendications précédentes, dans laquelle le polymère insaturé est un homopolymère de LDPE insaturé ou un copolymère de LDPE avec un ou plusieurs comonomères polyinsaturés, de préférence, un ou plusieurs diènes, notamment, un polymère sous la forme (1) d'une poudre de polymère, (2) de pastilles de polymère ou (3) d'un mélange d'une masse fondue de polymères, ladite (1) poudre de polymère ou, de préférence, (2) lesdites pastilles de polymère étant éventuellement contenues dans un récipient.

**17.** Composition de polymère modifiée, dans laquelle la composition de polymère selon l'une quelconque des revendications précédentes est réticulée par initiation d'une réaction radicalaire au sein de la composition de polymère.

**18.** Câble réticulable qui comprend un conducteur qui est entouré d'une ou de plusieurs couches, par exemple, une couche d'isolation, une couche de chemisage et/ou une couche semiconductrice comprenant une composition de polymère selon l'une des revendications 1 à 16 ou câble réticulé, dans lequel ladite composition de polymère a été réticulée par initiation d'une réaction radicalaire au sein de la composition de polymère, de préférence,

- un câble basse tension comprenant un conducteur entouré d'une couche d'isolation et éventuellement, d'une couche de chemisage, dans lequel ladite couche d'isolation comprend une composition de polymère selon l'un des revendications 1 à 16 ; ou
- un câble électrique comprenant un conducteur électrique entouré d'une ou de plusieurs couches comprenant au moins une couche semiconductrice intérieure, une couche d'isolation et une couche semiconductrice extérieure, dans cet ordre, et éventuellement entouré d'une couche de chemisage, dans lequel au moins une desdites couches comprend, une composition de polymère selon les revendications 1 à 16.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9308222 A
- WO 9635732 A


### Non-patent literature cited in the description


- **Milas, N.A. ; Surgenor, D.M.** *J. Am. Chem. Soc.,* 1946, 643-644
- **Hey, D.H. ; Stirling, C.J.M. ; Williams, G.H.** *J. Chem. Soc.,* 1957, 1054-1058
- **Smith, M.B.** Organic Synthesis. The McGraw-Hill Companies Inc, 2002
- Encyclopedia of Polymer Science and Engineering. 1986, vol. 6, 383-410